# EUROPEAN PATENT SPECIFICATION

(11) **EP 2 585 595 B1**
(45) Date of publication and mention of the grant of the patent: **16.08.2017**
(21) Application number: 11731485.6
(22) Date of filing: 23.06.2011
(51) Int. Cl.: C12N 15/113

(54) **RNA MOLECULES AND USES THEREOF**
RNA-MOLEKÜLE UND IHRE VERWENDUNG
MOLÉCULES D'ARN ET LEURS UTILISATIONS

(30) Priority: 23.06.2010 GB 201010557
(43) Date of publication of application: 01.05.2013
(73) Proprietor: Mina Therapeutics Limited, Floor 6 London EC2Y 5EB (GB)
(72) Inventor: SÆTROM, Pål, NO-7023 Trondheim (NO)
(74) Representative: Hoffmann Eitle
(86) International application number: PCT/GB2011/051185
(87) International publication number: WO 2011/161460

(56) References cited:
- WO-A1-2010/047216
- WO-A2-2009/046397
- KEVIN V. MORRIS ET AL: "Bidirectional Transcription Directs Both Transcriptional Gene Activation and Suppression in Human Cells", PLOS GENETICS, vol. 4, no. 11, 1 January 2008 (2008-01-01), page E1000258, XP055008804, ISSN: 1553-7390, DOI: 10.1371/journal.pgen.1000258
- LI LONG-CHENG ET AL: "Small dsRNAs induce transcriptional activation in human cells", PROCEEDINGS OF THE NATIONAL ACADEMY OF SCIENCES OF USA, NATIONAL ACADEMY OF SCIENCE, WASHINGTON, DC; US, vol. 103, no. 46, 14 November 2006 (2006-11-14), pages 17337-17342, XP002589998, ISSN: 0027-8424, DOI: 10.1073/PNAS.0607015103 [retrieved on 2006-11-03]
- SCHWARTZ J C ET AL: "Antisense transcripts are targets for activating small RNAs", NATURE STRUCTURAL & MOLECULAR BIOLOGY,, vol. 15, no. 8, 6 July 2008 (2008-07-06), pages 842-848, XP009109339, ISSN: 1545-9985, DOI: 10.1038/NSMB.1444
- PLACE ROBERT F ET AL: "MicroRNA-373 induces expression of genes with complementary promoter sequences", PROCEEDINGS OF THE NATIONAL ACADEMY OF SCIENCES OF USA, NATIONAL ACADEMY OF SCIENCE, WASHINGTON, DC; US, vol. 105, no. 5, 5 February 2008 (2008-02-05), pages 1608-1613, XP002548636, ISSN: 0027-8424, DOI: 10.1073/PNAS.0707594105 [retrieved on 2008-01-28]
- NA XU ET AL: "MicroRNA-145 Regulates OCT4, SOX2, and KLF4 and Represses Pluripotency in Human Embryonic Stem Cells", CELL, vol. 137, no. 4, 1 May 2009 (2009-05-01), pages 647-658, XP055008854, ISSN: 0092-8674, DOI: 10.1016/j.cell.2009.02.038
- JANOWSKI B A ET AL: "Activating gene expression in mammalian cells with promoter-targeted duplex RNAs", NATURE CHEMICAL BIOLOGY, NATURE PUBLISHING GROUP, NEW YORK, NY, US, vol. 3, no. 3, 28 January 2007 (2007-01-28), pages 166-173, XP003026584, ISSN: 1552-4450, DOI: 10.1038/NCHEMBIO860
- ELMÉN J ET AL: "Antagonism of microRNA-122 in mice by sistemically administered LNA-antimiR leads to up-regulation of a large set of predicted target mRNAs in the liver", NUCLEIC ACIDS RESEARCH, OXFORD UNIVERSITY PRESS, SURREY, GB, vol. 36, no. 4, 23 December 2007 (2007-12-23), pages 1153-1162, XP002533568, ISSN: 0305-1048, DOI: 10.1093/NAR/GKM1113
- Y. TIAN ET AL: "MicroRNA-10b Promotes Migration and Invasion through KLF4 in Human Esophageal Cancer Cell Lines", JOURNAL OF BIOLOGICAL CHEMISTRY, vol. 285, no. 11, 12 March 2010 (2010-03-12), pages 7986-7994, XP055008850, ISSN: 0021-9258, DOI: 10.1074/jbc.M109.062877
- SARA NAPOLI ET AL: "Promoter-specific transcriptional interference and c-myc gene silencing by siRNAs in human cells", THE EMBO JOURNAL, vol. 28, no. 12, 17 June 2009 (2009-06-17) , pages 1708-1719, XP055009047, ISSN: 0261-4189, DOI: 10.1038/emboj.2009.139
- KALANTARI ROYA ET AL: "Regulation of mammalian transcription and splicing by Nuclear RNAi", NUCLEIC ACIDS RESEARCH, vol. 44, no. 2, January 2016 (2016-01), pages 524-537, ISSN: 0305-1048(print)

## Description

The present invention relates to short RNA molecules capable of modulating the expression of target genes and to their design, synthesis and uses.

RNA interference (RNAi) is an important gene regulatory mechanism that causes sequence-specific down-regulation of target mRNAs. RNAi is mediated by "interfering RNA" (iRNA); an umbrella term which encompasses a variety of short double stranded RNA (dsRNA) molecules which function in the RNAi process.

Exogenous dsRNA can be processed by the ribonuclease protein Dicer into double-stranded fragments of 19 to 25 base pairs with several unpaired bases on each 3' end forming a 3' overhang. These short double-stranded fragments are termed small interfering RNAs (siRNAs) and these molecules effect the down-regulation of the expression of target genes.

Since the elucidation of their function, siRNAs have been used as tools to down-regulate specific genes. They can give transient suppression or, when stably integrated as short hairpins RNAs (shRNAs), stable suppression. siRNAs and shRNAs have been used widely in "knockdown" or "loss of function" experiments, in which the function of a gene of interest is studied by observing the effects of the decrease in expression of the gene. RNAi is considered to have potential benefits as a technique for genomic mapping and annotation. Attempts have also been made to exploit RNA interference in therapy.

A protein complex called the RNA-induced silencing complex (RISC) incorporates one of the siRNA strands and uses this strand as a guide to recognize target mRNAs. Depending on the complementarity between guide RNA and mRNA, RISC then destroys or inhibits translation of the mRNA. Perfect complementarity results in mRNA cleavage and destruction and as result of the cleavage the mRNA can no longer be translated into protein. Partial complementarity - particularly with sites in the mRNA's 3' untranslated region (UTR) - results in translational inhibition. RNAi is conserved in most eukaryotes and can, by introducing exogenous siRNAs, be used as a tool to down-regulate specific genes.

Recently it has been discovered that although RISC primarily regulates genes post transcription, RNAi can also modulate gene transcription itself. In fission yeast, small RNAs regulate chromatin through homologues of the RISC complex. The RNA-loaded RISC complexes apparently bind non-coding RNAs (ncRNA) and thereby recruit histone-modifying proteins to the ncRNAs' loci. Plants, flies, nematodes, ciliates, and fungi also have similar mechanisms. In mammals, much of the exact mechanism remains unclear, but it is believed that short RNAs regulate transcription by targeting for destruction transcripts that are sense or antisense to the regulated RNA and which are presumed to be non-coding transcripts. Destruction of these non-coding transcripts through RNA targeting has different effects on epigenetic regulatory patterns depending on the nature of the RNA target. Destruction of ncRNA targets which are sense to a given mRNA results in transcriptional repression of that mRNA, whereas destruction of ncRNA targets which are antisense to a given mRNA results in transcriptional activation of that mRNA. By targeting such antisense transcripts, RNAi can therefore be used to up-regulate specific genes. Short RNA molecules which lead to the up-regulation of target genes are termed short activating RNA molecules (saRNAs).

Known methods of up-regulating a target gene by use of saRNAs involve the detection of an RNA transcript which is antisense to the target gene of interest and designing short RNA molecules which down-regulate the identified transcript. Target antisense RNA transcripts are identified from databases of known transcripts or ESTs within the genomic region around the locus of the gene of interest. Alternatively, Reverse Transcriptase PCR (RT-PCR), a well-known tool for identifying RNA, is used to identify potential target RNA transcripts.

For instance, US 2009/0092988 discloses a method of selectively modulating expression of a target gene in the genome of a mammalian cell comprising determining the presence of an encoded antisense transcript and contacting the transcript with an exogenous double-stranded RNA which is complementary to a portion of the transcript.

The inventor has provided a novel algorithm/method designing short activating RNA molecules which up-regulate a target gene..This design method finds wide applicability throughout the genome including genes involved in pluripotency.

Kruppel-like factor 4 (gut) (KLF4) is a transcription factor that is important for maintaining embryonic stem (ES) cells. Ectopic expression of KLF4 and the three other transcription factors POU5F1 (also called OCT3/4), SOX2, and MYC has been shown to reprogram mouse and human fibroblasts into induced pluripotent stem (iPS) cells. A recent study of the transcriptional network of ES cells indicate that KLF4 is a master regulator that controls the expression of other pluripotency factors, including POU5F1, SOX2, MYC, and NANOG [Kim et al. (2008) Cell 132: 1049-1061].

The development of induced pluripotent stem cells (iPSC) has been a milestone in the understanding of the stem cell field. iPSC technology relies on up-regulation of the four genes KLF4, POU5F1 (Oct3/4), SOX2, and MYC, or, in certain cells, up-regulation of some of these four genes and other pluripotency factors such as NANOG and LIN28. This was realised by genetic introduction of these genes to non-embryonic stem cells. It has been suspected that manipulating KLF4 expression may therefore be an effective method for controlling stem cells.

Non-embryonic cells such as fibroblasts can be induced to a pluripotent stem cell state by activation of the above mentioned genes. From a therapeutic perspective, the main advantage of iPSCs is that the patient's own fibroblasts can be reprogrammed to produce stem cells, which eliminates the need for immunosuppressive drugs or the matching of histocompatability genes.

Current methods of up-regulating the expression of pluripotency factors require the introduction of extra copies of the genes (known in the field as stemness genes), either by using viruses to introduce extra copies of the genes into the host genome or by introducing plasmids that express extra copies of the stemness genes. Thus, for up-regulation of KLF4 and other stemness factors, invasive transient transfection or stable viral transduction of expression vectors into cells is currently required. The current methods involve the non-transient application of up-regulatory agents, A limitation of these methods is that the effects are similarly non-transient, i.e. the induced stem cells can not be expanded and then reprogrammed to differentiate.

The present inventor has developed new short RNA molecules which achieve up-regulation or down-regulation of target genes and which overcome the problems associated with the above methods of the prior art. In particular, the molecules of the present disclosure are less invasive and their effects are transient. The present disclosure therefore includes novel short RNA molecules which target RNA transcripts in the host cell in order to modulate target genes and in particular target genes which are pluripotency-inducing genes or genes which cause differentiation. The short RNAs disclosed herein are smaller molecules than the expression vectors of the prior art and so are therefore less invasive, however, the fact that the molecules of this disclosure use the host's own regulatory systems to modulate genes is also less invasive than introducing into the host extra copies of the genes.

The short RNAs of the present disclosure can up-regulate mRNA and protein levels of the target genes. The algorithm design method disclosed herein leads to the design of short activating RNA molecules (saRNAs) which up-regulate the expression of a target gene. Demonstrated herein is the up-regulation of a disparate selection of genes which are up-regulated by saRNA molecules designed using the method of the present invention, including KLF4, MYC, POU5F1, SOX2, BCL2 and IL-8.

The KLF4-, MYC-, POU5F1- and SOX2-activating RNAs of the present disclosure are an effective, non-invasive, and safe alternative for expanding hematopoietic stem cells to be used in regenerative medicine.

A major advantage of the present invention is that it concerns the transient application of gene-activating small RNAs, whose effects are also transient. This permits the induced stem cells to be expanded and subsequently re-programmed to differentiate. The present invention provides a method of producing a single-stranded, and not double stranded, short RNA molecule to increase the expression of a target gene in a cell through the down-regulation of a non-coding RNA transcript, said method comprising the steps of:
a) obtaining the nucleotide sequence of the coding strand of the target gene between 200 nucleotides upstream of the gene's transcription start site and 200 nucleotides downstream of the gene's transcription start site;
b) determining the reverse complementary RNA sequence to the nucleotide sequence determined in step a);
c) designing a single-stranded short RNA molecule which is from 16 nucleotides tc 30 nucleotides in length and which is the reverse complement or has at least 95% sequence identity with the reverse complement of a region of the sequence determined in step b); and
d) synthesising the single-stranded short RNA molecule, and not a double-stranded short RNA molecule, wherein the synthesised single-stranded short RNA molecule comprises non-standard nucleotides;
wherein said method does not include a step in which the existence of said non-coding RNA transcript is determined.
Further aspects of the invention are defined in the claims.

Also disclosed herein is a method of designing a short RNA molecule to increase the expression of a target gene in a cell through the down-regulation of a non-coding RNA transcript, said method comprising the steps of:
a) obtaining the nucleotide sequence of the coding strand of the target gene, at least between 200 nucleotides upstream of the gene's transcription start site and 200 nucleotides downstream of the gene's transcription start site;
b) determining the reverse complementary RNA sequence to the nucleotide sequence determined in step a); and
c) designing a short RNA molecule which is the reverse complement or has at least 80% sequence identity with the reverse complement of a region of the sequence determined in step b);
wherein said method does not include a step in which the existence of said non-coding RNA transcript is determined.

The disclosure also includes a method of designing a short RNA molecule to increase the expression of a target gene in a cell through the down-regulation of a non-coding RNA transcript, said method comprising the steps of:
i) obtaining the nucleotide sequence of the coding strand of the target gene, at least between 200 nucleotides upstream of the gene's transcription start site and 200 nucleotides downstream of the gene's transcription start site; and
ii) designing a short RNA molecule which has at least 80% sequence identity to a region of the sequence determined in step i), wherein for the purpose of determining sequence identity a uracil nucleotide in the short RNA molecule is considered identical to a thymine residue in the region of the sequence determined in step i);
wherein said method does not include a step in which the existence of said non-coding RNA transcript is determined.

The terms "method of designing" and "design method" are used interchangeably herein with the terms "algorithm for designing" and "algorithm".

The "coding strand" of a gene is the strand which contains the coding sequence for the gene's mRNA. The "template strand" of a gene is the strand which does not contain the coding sequence for the gene's mRNA but is actually read by the RNA polymerase.

As used herein, the term "RNA" means a molecule comprising at least one ribonucleotide residue. By "ribonucleotide" is meant a nucleotide with a hydroxyl group at the 2' position of a beta-D-ribo-furanose moiety. The terms include double stranded RNA, single stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of the RNA or internally, for example at one or more nucleotides of the RNA. Nucleotides in the RNA molecules of the present disclosure can also comprise non-standard nucleotides, such as non-naturally occurring nucleotides or chemically synthesized nucleotides or deoxynucleotides. These altered RNAs can be referred to as analogs or analogs of naturally-occurring RNA.

The term "double stranded RNA" or "dsRNA" as used herein refers to a ribonucleic acid duplex, including but not limited to, endogenous and artificial siRNAs, short hairpin RNAs (shRNAs) and micro RNAs (miRNAs).

The term "short interfering RNA" or "siRNA" as used herein refers to a nucleic acid molecule capable of modulating gene expression through RNAi via sequence-specific-mediated cleavage of one or more target RNA transcripts. Typically in RNAi the RNA transcript is mRNA and so cleavage of this target results in the down-regulation of gene expression. In this invention however, up-regulation or down-regulation of the target gene can be achieved by cleavage of RNA transcripts which are antisense or sense to the target gene of interest respectively. Such short RNA molecules are termed short (or small) activating saRNA molecules (saRNAs) when they enhance gene expression and they may have the same structural features as other short RNA molecules, such as siRNAs.

siRNAs are double-stranded RNA molecules, typically of 19 to 25 base pairs in length with several unpaired bases on each 3' end forming a 3' overhang. siRNAs contain one strand with a sequence of perfect or near perfect complementarity to a region of a target RNA transcript. A protein complex known as the RNA-induced silencing complex (RISC), incorporates this strand of the siRNA duplex (the guide strand) and uses it as a template to recognize the target RNA transcript. RISC is then involved in the cleavage of the target RNA transcript with perfect or near-perfect complementarity to the incorporated strand. The other strand of the siRNA molecule, which does not possess complementarity to a region of the target RNA transcript is termed the passenger strand.

Single stranded RNA molecules which are not siRNA molecules but which are capable of down-regulating a target RNA transcript to which they have perfect or near-perfect complementarity by RISC-associated cleavage, are said to have siRNA-like activity. The short RNA molecules designed by the method of the present invention have this activity.

By "activation" or "up-regulation" of a gene is meant an increase in the level of expression of a gene(s), or levels of the polypeptide(s) encoded by a gene or the activity thereof, or levels of the RNA molecule(s) transcribed from a gene above that observed in the absence of the short RNA molecules designed by the method of the present invention.

The short RNA molecules designed by the method disclosed herein effectively and specifically up-regulate a target gene in a cell, i.e. they increase the expression of that target gene, through the down-regulation of an RNA transcript which is antisense to a genomic sequence on the coding strand of the target gene. Without wishing to be bound by theory, it is believed that the antisense RNA transcript represses the expression of the target gene and that the short RNA molecules designed by the present algorithm up-regulate the target gene by down-regulating the down-regulatory antisense RNA transcripts. As mentioned above, this can be achieved by the short RNA having a high degree of complementarity to a sequence within the antisense RNA transcript.

However, the algorithms herein do not require the identification of any RNA transcripts which are antisense to the target gene. The present inventors found, surprisingly, that if the nucleotide sequence of the coding strand of the gene in the region 200 nucleotides upstream of the gene's transcription start site to 200 nucleotides downstream of the gene's transcription start site is obtained, i.e. determined by sequencing or found on a database, and the reverse complementary RNA sequence to that region is determined, then short RNA molecules which are in turn the reverse complement or have at least 80% sequence identity with the reverse complement of that latter sequence can be used to up-regulate the target gene. Whereas algorithms/ methods for the design of short activating RNA molecules in the prior art require the determination of the existence of antisense RNA transcripts to target, either from databases or via RT-PCR, the methods of the present invention do not have this requirement. As a result, the methods of the present invention provide a far quicker, cheaper and more efficient means of producing short activating RNA molecules against any target gene of interest. The realization that it is not necessary to confirm the presence or identity of antisense RNA transcripts before saRNAs can be designed led the inventors to the methods of the present invention.

Thus, the methods of the present invention do not include a step in which the existence of said non-coding RNA transcript (i.e. the target transcript to be downregulated) is determined. The prior art methods of designing saRNAs determine the existence of a non-coding RNA transcript target. "Determination of existence" means either searching databases of ESTs and/or antisense transcripts around the locus of the target gene to identify a suitable target transcript, or using RT PCR or any other known technique to confirm the physical presence of a target antisense RNA transcript in a cell.

In step a) or step i) of the methods of the present disclosure, the nucleotide sequence of the coding strand of the gene, at least between 200 nucleotides upstream of the gene's transcription start site and 200 nucleotides downstream of the gene's transcription start site, is obtained. This sequence can be obtained by reference to databases of genetic sequences, which are well-known to the skilled man, or by sequencing the sequence. Tools for sequencing a gene of interest are also well-known by those of ordinary skill in the art.

Preferably, step a) and step i) of the methods above comprise obtaining the nucleotide sequence of the coding strand of the target gene, at least between 300 nucleotides upstream of the gene's transcription start site and 300 nucleotides downstream of the gene's transcription start site. More preferably, step a) and step i) of the methods above comprise obtaining the nucleotide sequence of the coding strand of the target gene, at least between 500 nucleotides upstream of the gene's transcription start site and 500 nucleotides downstream of the gene's transcription start site. Still more preferably, step a) and step i) of the methods above comprise obtaining the nucleotide sequence of the coding strand of the target gene, at least between 1000 nucleotides upstream of the gene's transcription start site and 1000 nucleotides downstream of the gene's transcription start site

Viewed in one way, the method further comprises step b) above, in which the reverse complementary RNA sequence to the nucleotide sequence determined in step a) is determined. Unlike the methods of the prior art, the existence of this RNA sequence is not determined, i.e. the RNA sequence determined is a putative, theoretical sequence. The step does not include making reference to any database or using any known technique to determine the actual existence of an RNA transcript with the determined sequence.

Step c) above requires the design of a short RNA molecule which is the reverse complement or has at least 80% sequence identity with the reverse complement of a region of the sequence determined in step b). Preferably, the short RNA molecule is the reverse complement or has at least 85%, more preferably, 90%, still more preferably 95% sequence identity with the reverse complement of a region of the sequence determined in step b). If a first sequence is the reverse complement of a second sequence then it has perfect or near-perfect complementarity to that second sequence in the reverse direction.

By "complementarity" and "complementary" are meant that a first nucleic acid can form hydrogen bond(s) with a second nucleic acid for example by Watson-Crick base pairing. A nucleic acid which can form hydrogen bond(s) with another nucleic acid through non-Watson-Crick base pairing also falls within the definition of having complementarity. A percent complementarity indicates the percentage of residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). Preferably the method of the present disclosure includes a step of designing a short RNA molecule with any such degree of complementarity over its entire length to a region of the RNA sequence determined in step b). The short RNA molecule is preferably at least 85%, 90% or 95%, most preferably 100% complementary over its entire length to a region of the RNA sequence determined in step b).

The short RNA will have no more than 5, preferably no more than 4 or 3, more preferably no more than 2, still more preferably no more than 1, most preferably no mismatches with the sequence of the region of the RNA transcript determined in step b) to which it is complementary. In this scenario, a "mismatch" is when at a given position within the two sequences, the nucleotides present in the sequences are not complementary.

The determination of the degree of complementarity of two or more sequences can be performed by any method known in the art. Preferably, the method used is that set out in Hossbach *et al.* (*supra*)*.* In accordance with this method, the Perl script accessible at http://www.mpibpc.mpa.de/groups/luehrmann/siRNA is used.

"Perfectly complementary" or "perfect complementarity" means that all sequential residues of a first nucleic acid sequence will form hydrogen bonds with the same number of sequential residues in a second nucleic acid sequence. "Near-perfect" complementary means that essentially all sequential residues of a first nucleic acid sequence will form hydrogen bonds with the same number of sequential residues in a second nucleic acid sequence, however, due to the fact that the first nucleic acid is prepared by an imperfect process such as transcription or a molecular biological process involving the use of biological molecules, the first sequence may not be 100% complementary to the second sequence. However, the number of residues in the first sequence incapable of forming hydrogen bonds with the corresponding residues in the second sequence is sufficiently low that the two nucleic acid sequences are still bonded via hydrogen bonds to the extent required for the desired purpose. Typically, "near-perfect complementarity" means that a first nucleic acid sequence has at least 95% complementarity with a second nucleic acid sequence. Preferably the short RNA molecule has near-perfect, more preferably perfect complementarity over its entire length to the a region of the RNA sequence determined in step b).

The short RNA molecule does not need to be the reverse complement of the region of the sequence determined in step b), instead it may have a degree of sequence identity with the reverse complement of the region of the sequence determined in step b). By "identity", "identical" or "sequence identity" is meant that a first nucleic acid is identical in sequence to a second nucleic acid sequence. A percent identity indicates the percentage of residues in a first nucleic acid molecule that are identical to a second nucleic acid sequence (e.g., 5, 6, 7, 8, 9, 10 out of 10 being 50%, 60%, 70%, 80%, 90%, and 100% identical). Preferably the method of the present disclosure includes a step of designing a short RNA molecule with this degree of sequence identity over its entire length with the reverse complement of the region of the sequence determined in step b). The short RNA molecule has preferably at least 85%, 90% or 95%, most preferably 100% sequence identity over its entire length with the reverse complement of the region of the sequence determined in step b). The short RNA will have no more than 5, preferably no more than 4 or 3, more preferably no more than 2, still more preferably no more than 1, most preferably no "mismatches" with the sequence of the reverse complemt of the region of the sequence determined in step b). In this scenario a "mismatch" is when at a given position within the two sequences, the nucleotides present in the sequences are not complementary.

Preferably, the short RNA molecule is the reverse complement or has at least 80% sequence identity or any other sequence identity recited herein with the reverse complement of a region of the sequence determined in step b) which is itself the reverse complement of a region of the gene's coding strand comprising the transcription start site. In other words, preferably the short RNA molecule is the reverse complement or has a degree of sequence identity with the reverse complement of a region of the sequence determined in step b), said region including within it the reverse complement of the gene's transcription start site.

Alternatively viewed, the method of the present disclosure comprises designing a short RNA molecule which is identical to a region of the sequence determined in step i). By "identity", "identical" or "sequence identity" is meant that a first nucleic acid is identical in sequence to a second nucleic acid sequence. A percent identity indicates the percentage of residues in a first nucleic acid molecule that are identical to a second nucleic acid sequence (e.g., 5, 6, 7, 8, 9, 10 out of 10 being 50%, 60%, 70%, 80%, 90%, and 100% identical). Preferably the method
includes a step of designing a short RNA molecule with this degree of identity over its entire length to a region of the sequence determined in step i). The short RNA molecule has preferably at least 85%, 90% or 95%, most preferably 100% sequence identity over its entire length with the region of the sequence determined in step i). The short RNA will have no more than 5, preferably no more than 4 or 3, more preferably no more than 2, still more preferably no more than 1, most preferably no "mismatches" with a region of the sequence determined in step i). In this scenario a "mismatch" is when at a given position within the two sequences, the nucleotides present in the sequences are not identical.

Preferably, the short RNA molecule is identical to a region of the coding strand of the gene which includes the gene's transcription start site.

"Perfect identity" or "perfectly identical" means that all sequential residues of a first nucleic acid sequence are identical to the same number of sequential residues in a second nucleic acid sequence. "Near-perfect" identity means that essentially all sequential residues of a first nucleic acid sequence are identical to the same number of sequential residues in a second nucleic acid sequence, however, due to the fact that the first nucleic acid is prepared by an imperfect process such as transcription or a molecular biological process involving the use of biological molecules, the first sequence may not be 100% identical to the second sequence. However, the number of residues in the first sequence which are not identical to the corresponding residues in the second sequence is sufficiently low that the two nucleic acid sequences are still sufficiently identical for the given purpose. Typically, "near-perfect identity" means that a first nucleic acid sequence has at least 95% identity with a second nucleic acid sequence.

Sequence alignments and percent identity or percent complementarity calculations may be determined using any method or tool known in the art including but not limited to the Megalign program of the LASARGENE bioinformatics computing suite (DNASTAR Inc., Madison, WI), the Clustal V method of alignment (Higgins and Sharp (1989) CABIOS. 5:151-153) and the BLAST 2.0 suite of programs. Software for performing BLAST analyses is publicly available, e.g., through the National Center for Biotechnology Information. The skilled man will be able to set the parameters of these tools to suit his desired purpose.

When assessing the identity or complementarity of a first and second nucleic acid sequence wherein one sequence is a DNA sequence and the other is an RNA sequence, it must be borne in mind that RNA sequences comprise uracil whereas DNA sequences would comprise thymine instead. Therefore, in these instances when assessing sequence identity, a uracil residue is considered to be identical to a thymine residue and when assessing complementarity a uracil residue is considered to be complementary to/capable of forming hydrogen bonds with an adenine residue.

The size of the 'region' corresponds to the size of the short RNA molecule and preferred sizes of these molecules are defined herein. The region and the short RNA molecule will typically be less than 100 nucleotides in length, preferably less than 50 nucleotides in length, but at least 12 nucleotides in length, more preferably 16 to 30 nucleotides in length.

Optionally, step c) and step ii) above comprise further steps which lead to the design of short RNA molecules with particularly desirable structural and/or functional properties. These properties will be discussed below along with the tools required for designing short RNA molecules with these properties.

Optional and preferred features of the short RNA molecules designed by the method disclosed herein will now be discussed. The method preferably comprises a step of designing short RNA molecules with each, any and all of these preferred features.

Preferably the "short" RNA molecules designed by the method disclosed herein are from 16 nucleotides to 30 nucleotides in length, more preferably 19 to 30 nucleotides in length, still more preferably 19 to 25 or 19 to 23 nucleotides in length, most preferably 19 or 21 nucleotides in length.

The short RNA molecule designed are single stranded.

Various tools for the design and analysis of short RNA molecules are well-known, which permit one of ordinary skill in the art to determine those RNA molecules which can achieve effective and specific down-regulation of a target RNA transcript, i.e. a target antisense transcript. Established methods include, for example, the GPboost and Reynolds algorithms (PMIDs: 15201190, 14758366). In addition, the ability of a short RNA to cause effective down-regulation of a target RNA can be evaluated using standard techniques for measuring the levels of RNA or protein in cells. For example, a short RNA of the invention can be delivered to cultured cells, and the levels of target RNA can be measured by techniques including but not limited to Northern blot or dot blotting techniques, or by quantitative RT-PCR.

Preferably the short RNAs designed possess none of the motifs aaaa, cccc, gggg, or tttt. Preferably the short RNAs have a GC-percentage of at least 20% and no more than 75 %, i.e. between 20% and 75%, preferably between 20% and 55%. The short RNAs of the above methods are ideally thermodynamically stable duplexes, in which case the GC percentage of each strand is at least 25% and no more than 75 %, i.e. between 25% and 75%, preferably between 20% and 55%, more preferably between 20% and 50%.

Tools and algorithms for determining whether or not RNAs possess the motifs aaaa, cccc, gggg or tttt and for determining the percentage GC content of the molecules/strands are well known to the skilled artisan. Such tools include those described and referenced in Saetrom and Snove, (2004) Biochem Biophys Res Commun 321: 247-253 and Vert et al., (2006) BMC Bioinformatics 7: 520 (17 pages).

Short RNAs can induce down-regulation of non-target transcripts that have a limited number of mismatches to the short RNA strand which is incorporated into the RISC protein complex. This reduces the efficiency of the short RNA molecule and is therefore not desired. Consequently, short RNA molecules should have limited complementarity to transcripts other than the intended target to prevent unintended off-target effects. The probability of a short RNA candidate having cleavage-based off-target effects is a function of its complementarity to non-target RNA sequences and can be determined by any known method in the art. Optionally, an ungapped Smith-Waterman method (TF Smith & MS Waterman (1981) Journal of molecular biology 147: 195-197) can be used to screen the candidate short RNA against the Ensembl (Flicek, P., et al. (2008) Ensembl 2008. Nucleic Acids Res 36: D 707-714) human transcriptome database (Snøve, O., Jr., et al. (2004) Biochem Biophys Res Commun 325: 769-773) to identify a short RNA's potential off-target transcripts. Alternatively, the short RNA can be screened against a population of chosen RNA sequences, for example a selection of GenBank sequences, which do not encompass the entire Ensembl human transcriptome database. Alternatively a Hamming distance measure can be used.

Preferably, the short RNA molecules have more than two mismatches to identified off-target transcripts Alternatively viewed, preferably the short RNA molecules have a Hamming distance of 2 or greater to all potential off-target transcripts. If the short RNA is part of a double stranded molecule then preferably both strands satisfy this requirement.

Optionally, the short RNA molecules have characteristics in common with known highly effective standard siRNAs. Preferably, the short RNA, or if part of a double-stranded molecule one or both strands of the short RNA, has a GPboost score of more than 0.1. GPboost is a known genetic programming-based prediction system of siRNA efficacy and the methods used for determining the GPboost score of siRNA strands is disclosed in "Predicting the efficacy of short oligonucleotides in antisense and RNAi experiments with boosted genetic programming", Pål Saetrom (2004) Bioinformatics 20(17): 3055-3063.

Alternatively or in addition, the short RNA molecules possess specific sequence features which are associated with highly effective siRNAs. The algorithm described by Reynolds [Reynolds et al. (2004) Nature biotechnology 22(3):326-330] permits the determination of whether or not short RNAs possess sufficient features of this type. One of ordinary skill in the art would be able to define and refine his threshold for his particular purpose.

Optionally, the short RNA molecules contain position-specific sequence motifs which are associated with highly effective siRNAs siRNA efficacy prediction algorithms are well-known in the art and motifs which are associated with highly-effective siRNAs are discussed in Saetrom and Snove, (2004) Biochem Biophys Res Commun 321: 247-253.

Optionally, support vector machines (SVMs) can be used to provide an additional measure of the likelihood of a given short RNA sequence being effective in down-regulating a target transcript. A support vector machine (SVM) is a concept in computer science for a set of related supervised learning methods that analyze data and recognize patterns, used for classification and regression analysis. The standard SVM takes a set of input data and predicts, for each given input, which of two possible classes the input is a member of, which makes the SVM a non-probabilistic binary linear classifier. Given a set of training examples, each marked as belonging to one of two categories, an SVM training algorithm builds a model that assigns new examples into one category or the other. An SVM model is a representation of the examples as points in space, mapped so that the examples of the separate categories are divided by a clear gap that is as wide as possible. New examples are then mapped into that same space and predicted to belong to a category based on which side of the gap they fall on. Any known SVM can be used in the design of saRNA molecules disclosed herein. Particularly suitable SVMs are described in Sætrom (2004) Bioinformatics 20 (17): 3055-3063*.* Preferably, short RNA molecules are selected when they have a SVM score of greater than 0.

Preferably the short RNA molecule is capable of direct entry into the RNAi machinery of a cell or is capable of being processed by Dicer before entry into the RNAi machinery of a cell. Methods of determining whether or not a short RNA molecule is capable of being processed by Dicer before entry into the RNAi machinery of a cell are well-known in the art, for instance in vitro Dicer assays such as that disclosed in Tiemann et al. (2010) RNA 16(6): 1275-1284 and Rose et al. (2005) Nucleic Acid Research 33(13):4140-4156.

If the short RNA molecule is part of a double stranded molecule (i.e. it is one strand of such a molecule) and if only that strand is capable of effectively and specifically down-regulating the target RNA transcript, then preferably that strand is preferentially loaded into RISC. The design of double-stranded RNA molecules in which one strand is preferentially loaded into RISC is within the competence of one of ordinary skill in the art. For instance, the 5' end of the strand of the short RNA molecule which targets the target RNA transcript can be made or selected to be less thermodynamically stable than the 5' end of the other strand. Preferably there is a large difference in duplex thermodynamic end stability such that the 5' end of the strand of the short RNA molecule which targets the target RNA transcript is less thermodynamically stable than the 5' end of the other strand. The absolute value of the difference in duplex thermodynamic end stability (ΔΔG) can be calculated in accordance with any method standard in the art. Optionally, the absolute value of the difference in duplex thermodynamic end stability is calculated by RNAfold (Hofacker et al., (2003) Nucleic Acids Research Vol. 31, No. 13, pp 3429-3431) by considering the 5 closing nucleotides at the ends of the duplex. Preferably the absolute value of the difference in duplex thermodynamic end stability as calculated by RNAfold is more than 0 kcal/mol, more preferably more than 1 kcal/mol, more preferably more than 3 kcal/mol.

Many standard tools for short RNA design, such as those described above, provide means for assessing this property of the molecules. For instance, double-stranded molecules can be selected if they have thermodynamic properties which favour the incorporation of one strand over the other into the RNAi machinery. Alternatively, the preferential loading of one strand can be achieved by using dsRNAs which contain RNA that differs from naturally-occurring RNA by the addition, deletion, substitution and/or alteration of one or more nucleotides. Such modifications are well-known to the skilled man and are discussed further below.

Dicer is a ribonuclease protein which cleaves exogenous dsRNA into double-stranded fragments of 19 to 25 base pairs with several unpaired bases on each 3' end forming a 3' overhang. The short RNAs used in the above-methods may be Dicer-substrate siRNAs (D-siRNAs). siRNAs designed as Dicer substrates can have increased potency compared to standard length siRNAs and shRNAs.

D-siRNAs are asymmetric siRNA-duplexes in which the strands are between 22 and 30 nucleotides in length. Typically, one strand (the passenger strand) is 22 to 28 nucleotides long, preferably 25 nucleotides long, and the other strand (the guide strand) is 24 to 30 nucleotides long, preferably 27 nucleotides long, such that the duplex at the 3' end of the passenger strand is blunt-ended and the duplex has an overhang on the 3'end of the guide strand. The overhang is 1 to 3 nucleotides in length, preferably 2 nucleotides. The passenger strand may also contain a 5' phosphate.

Typically in D-siRNAs, the two nucleotides at the 3' end of the passenger strand are deoxyribonucleic acids (DNAs) rather than ribonucleic acids (RNAs). The DNAs and the blunt-ended duplex ensure that the enzyme Dicer processes the duplex into a 21 mer duplex consisting of the 21 nucleotides at the 5' and 3' ends of the original D-siRNA's passenger and guide strands respectively.

Methods of extending standard 19mer siRNA molecules into D-siRNAs are well-known in the art, for instance as described in Hefner et al. (2008) J. Biomol. Tech. 19(4):231-237.

When extended to 27mer/25mer D-siRNAs, many siRNA molecules have an end structure where the predicted number of unpaired bases at the 3' end of the passenger strand is less than or equal to the predicted number of unpaired bases at the 5' end of the guide strand. Based on the structure of known miRNAs and the binding requirements of the Dicer PAZ-domain, this structure is most likely suboptimal for Dicer processing and so, while useful as siRNA molecules, such duplexes are less useful when extended to Dicer-substrate siRNA molecules. Therefore, preferably the short RNAs designed by the methods of the present invention do not possess such a structure and rather the predicted number of unpaired bases at the 3' end of the passenger strand is greater than the predicted number of unpaired bases at the 5' end of the guide strand.

Optionally the short RNA molecules designed by the methods herein can comprise modifications, i.e. RNA that differs from naturally-occurring RNA by the addition, deletion, substitution and/or alteration of one or more nucleotides. For instance, if the short RNA is part of a double stranded molecule, the two strands of the dsRNA molecule may be linked by a linking component such as a chemical linking group or an oligonucleotide linker with the result that the resulting structure of the dsRNA is a hairpin structure. The linking component must not block or otherwise negatively affect the activity of the dsRNA, for instance by blocking loading of strands into the RISC complex or association with Dicer. Many suitable chemical linking groups are known in the art. If an oligonucleotide linker is used, it may be of any sequence or length provided that full functionality of the dsRNA is retained. Preferably, the linker sequence contains higher amounts of uridines and guanines than other nucleotide bases and has a preferred length of about 4 to 9, more preferably 8 or 9 residues.

The short RNAs can be designed to contain modifications, provided that the modification does not prevent the RNA composition from serving as a substrate for Dicer. One or more modifications can be made that enhance Dicer processing of the dsRNA, that result in more effective RNAi generation, that support a greater RNAi effect, that result in greater potency per each dsRNA molecule to be delivered to the cell and/or that are helpful in ensuring dsRNA stability in a therapeutic setting.

Modifications can be incorporated in the 3'-terminal region, the 5'-terminal region, in both the 3 '-terminal and 5 '-terminal region or in some instances in various positions within the sequence. With the restrictions noted above in mind any number and combination of modifications can be incorporated into the RNA. Where multiple modifications are present, they may be the same or different. Modifications to bases, sugar moieties, the phosphate backbone, and their combinations are contemplated. Either 5'-terminus can be phosphorylated.

Short dsRNA molecules can be modified for Dicer processing by suitable modifiers located at the 3' end of the passenger strand, i.e., the dsRNA is designed to direct orientation of Dicer binding and processing. Suitable modifiers include nucleotides such as deoxyribonucleotides, dideoxyribonucleotides, acyclonucleotides and the like and sterically hindered molecules, such as fluorescent molecules and the like. Acyclonucleotides substitute a 2-hydroxyethoxymethyl group for the 2'-deoxyribofuranosyl sugar normally present in dNMPs. Other nucleotide modifiers could include 3'-deoxyadenosine (cordycepin), 3'-azido-3'- deoxythymidine (AZT), 2',3'-dideoxyinosine (ddl), 2',3'-dideoxy-3'-thiacytidine (3TC), 2',3'- didehydro-2',3'-dideoxythymidine (d4T) and the monophosphate nucleotides of 3'-azido-3'-deoxythymidine (AZT), 2',3'-dideoxy-3'-thiacytidine (3TC) and 2',3'-didehydro-2',3'-dideoxythymidine (d4T). Deoxynucleotides can be used as the modifiers. When nucleotide modifiers are utilized, 1-3 nucleotide modifiers, or 2 nucleotide modifiers are substituted for the ribonucleotides on the 3' end of the passenger strand. When sterically hindered molecules are utilized, they are attached to the ribonucleotide at the 3 ' end of the passenger strand. Thus, the length of the strand does not change with the incorporation of the modifiers. Optionally two DNA bases are substituted in the dsRNA to direct the orientation of Dicer processing. Optionally, two terminal DNA bases are located on the 3' end of the passenger strand in place of two ribonucleotides forming a blunt end of the duplex on the 5' end of the guide strand and the 3' end of the passenger strand, and a two-nucleotide RNA overhang is located on the 3 '-end of the guide strand. This is an asymmetric composition with DNA on the blunt end and RNA bases on the overhanging end.

Examples of modifications contemplated for the phosphate backbone include phosphonates, including methylphosphonate, phosphorothioate, and phosphotriester modifications such as alkylphosphotriesters, and the like. Examples of modifications contemplated for the sugar moiety include 2'-alkyl pyrimidine, such as 2'-O-methyl, 2'-fluoro, amino, and deoxy modifications and the like (see, e.g., Amarzguioui et al., 2003). Examples of modifications contemplated for the base groups include abasic sugars, 2-O-alkyl modified pyrimidines, 4-thiouracil, 5-bromouracil, 5-iodouracil, and 5-(3-aminoallyl)-uracil and the like. Locked nucleic acids, or LNA's, could also be incorporated. Many other modifications are known and can be used so long as the above criteria are satisfied.

The short RNAs designed by the methods herein can also comprise partially purified RNA, substantially pure RNA, synthetic RNA, or recombinantly produced RNA. Other possible alterations to the short RNAs include addition of non-nucleotide material to the end(s) of the short RNA or to one or more internal nucleotides of the short RNA; modifications that make the short RNA resistant to nuclease digestion (e.g., the use of 2'-substituted ribonucleotides or modifications to the sugar-phosphate backbone); or the substitution of one or more nucleotides in the short RNA with deoxyribonucleotides.

Steps c) and ii) may comprise initially generating a population of candidate saRNAs and then selecting from that population in a sequence of steps those which have a desired property or properties. The initial population may comprise some or every possible short RNA single-stranded sequence of a selected length or lengths which is complementary/identical to the sequence determined in step b) or step i), respectively.

A "target gene" or "gene of interest" is a gene whose expression is desired to be modulated. The term includes any nucleotide sequence, which may or may not contain identified gene(s), including, but not limited to, coding region(s), non-coding region(s), untranscribed region(s), intron(s), exon(s) and transgenes(s). The target gene can be a gene derived from a cell, an endogenous gene, a transgene or exogenous genes such as genes of a pathogen, which is present in the cell after infection thereof. The cell containing the target gene can be derived from or contained in any organism. A "target mRNA" sequence is an mRNA sequence derived from a target gene.

In a further aspect the present disclosure comprises a method of producing a short RNA molecule which comprises performing the method as defined anywhere herein and then synthesizing one or more of the RNA molecules designed by said method.

The short RNA molecules designed by the methods herein can be produced by any suitable method, for example synthetically or by expression in cells using standard molecular biology techniques which are well-known to the skilled artisan. For example, the short RNAs can be chemically synthesized or recombinantly produced using methods known in the art, such as the Drosophila in vitro system described in U.S. published application 2002/0086356 of Tuschl et al.*,* or the methods of synthesizing RNA molecules described in Verma and Eckstein (1998) Annu Rev Biochem 67: 99-134.

The short RNAs may be chemically synthesized using appropriately protected ribonucleoside phosphoramidites and a conventional DNA/RNA synthesizer. If the short RNAs are part of double-stranded RNAs then they can be synthesized as two separate, complementary RNA molecules, or as a single RNA molecule with two complementary regions. Commercial suppliers of synthetic RNA molecules or synthesis reagents include Proligo (Hamburg, Germany), Dharmacon Research (Lafayette, Colo., USA), Pierce Chemical (part of Perbio Science, Rockford, III., USA), Glen Research (Sterling, Va., USA), ChemGenes (Ashland, Mass., USA) and Cruachem (Glasgow, UK).

The short RNAs can also be expressed from recombinant circular or linear DNA plasmids using any suitable promoter. Suitable promoters for expressing short RNAs of the invention from a plasmid include, for example, the U6 or H1 RNA pol III promoter sequences and the cytomegalovirus promoter. Selection of other suitable promoters is within the skill in the art. The recombinant plasmids of the invention can also comprise inducible or regulatable promoters for expression of the short RNA in a particular tissue or in a particular intracellular environment.

The short RNAs expressed from recombinant plasmids can be isolated from cultured cell expression systems by standard techniques.

Selection of plasmids suitable for expressing short RNAs, methods for inserting nucleic acid sequences for expressing the short RNAs into the plasmid, and methods of delivering the recombinant plasmid to the cells of interest are within the skill in the art. See, for example Tuschl, T. (2002), Nat. Biotechnol. 20: 446-448 and Brummelkamp T R et al. (2002), Science 296: 550-553.

The short RNAs designed by the methods of the invention can also be expressed from recombinant viral vectors intracellularly *in vivo.* The recombinant viral vectors of the invention comprise sequences encoding the short RNAs of the invention and any suitable promoter for expressing the short RNA sequences. Suitable promoters include, for example, the U6 or H1 RNA pol III promoter sequences and the cytomegalovirus promoter. Selection of other suitable promoters is within the skill in the art.

Any viral vector capable of accepting the coding sequences for the dsRNAs molecule(s) to be expressed can be used, for example vectors derived from adenovirus (AV); adeno-associated virus (AAV); retroviruses (e.g, lentiviruses (LV), Rhabdoviruses, murine leukemia virus); herpes virus, and the like. The tropism of viral vectors can be modified by pseudotyping the vectors with envelope proteins or other surface antigens from other viruses, or by substituting different viral capsid proteins, as appropriate.

Selection of recombinant viral vectors, methods for inserting nucleic acid sequences for expressing the short RNA into the vector, and methods of delivering the viral vector to the cells of interest are within the skill in the art. See, for example, Dornburg R (1995), Gene Therap. 2: 301-310.

The present inventors have used the above-described method to design short activating saRNAs against a variety of target genes.

For example, the target gene may be a pluripotency-inducing gene. A pluripotency-inducing gene or "stemness gene" is a gene whose activation is known to be required for the induction or maintenance of pluripotency. Preferably the target gene is a gene selected from the group consisting of KLF4, POU5F1 (also called OCT3/4), SOX2 , MYC, NANOG and LIN28. More preferably the target gene is selected from the group consisting of KLF4, POU5F1 (also called OCT3/4), SOX2, and MYC. Most preferably the target gene is KLF4.

In a further aspect, the present disclosure includes a method of increasing the expression of a target gene in a cell through the down-regulation of a non-coding RNA transcript, said method comprising the steps of:
a) obtaining the nucleotide sequence of the coding strand of the target gene, at least between 200 nucleotides upstream of the gene's transcription start site and 200 nucleotides downstream of the gene's transcription start site;
b) determining the reverse complementary RNA sequence to the nucleotide sequence determined in step a);
c) designing a short RNA molecule which is the reverse complement or has at least 80% sequence identity with the reverse complement of a region of the sequence determined in step b); and
d) contacting the cell with said short RNA molecule,
wherein said method does not include a step in which the existence of said non-coding RNA transcript is determined.

The definitions and description above in relation to the methods of designing a short RNA molecule apply *mutatis mutandis* to this method of increasing the expression of a target gene in a cell.

The steps of the above methods of designing a short RNA molecule can be performed on a computer. Thus, the present disclosure also includes a computer-implemented method of designing a short RNA molecule to increase the expression of a target gene in a cell through the down-regulation of a non-coding RNA transcript, said method comprising the steps of:
a) obtaining the nucleotide sequence of the coding strand of the target gene, at least between 200 nucleotides upstream of the gene's transcription start site and 200 nucleotides downstream of the gene's transcription start site;
b) determining the reverse complementary RNA sequence to the nucleotide sequence determined in step a); and
c) designing a short RNA molecule which is the reverse complement or has at least 80% sequence identity with the reverse complement of a region of the sequence determined in step b);
wherein said method does not include a step in which the existence of said non-coding RNA transcript is determined.

The definitions and description above in relation to the methods of designing a short RNA molecule apply *mutatis mutandis* to this computer-implemented method of designing a short RNA molecule.

The methods of designing a short RNA molecule may be implemented, at least partially, using software e.g. computer programs. Thus, the present disclosure includes computer software specifically adapted to carry out any of the methods herein described when run on data processing means; a computer program element comprising computer software code portions for performing any of the methods herein described when the program element is run on data processing means, and a computer program comprising code means adapted to perform the steps of any of the methods herein described when the program is run on a data processing means.

The disclosure also extends to a computer software carrier comprising such software which when used to operate a processor, electronic device or system comprising data processing means causes, in conjunction with said data processing means, said processor, electronic device or system to carry out the steps of any of the methods described herein.

Thus, in a further aspect, the present disclosure includes one or more computer-readable media comprising computer-executable instructions to instruct a computing system to:
a) receive a nucleotide sequence of a coding strand of a target gene, at least between 200 nucleotides upstream of the gene's transcription start site and 200 nucleotides downstream of the gene's transcription start site;
b) determine a reverse complementary RNA sequence to the nucleotide sequence received in step a); and
c) output information to construct a short RNA molecule designed to increase expression of the target gene in a cell through the down-regulation of a non-coding RNA transcript wherein the short RNA molecule is the reverse complement or has at least 80% sequence identity with the reverse complement of a region of the sequence determined in step b);
wherein the instructions do not comprise instructions to call for determining existence of the non-coding RNA transcript.

Preferably, the one or more computer-readable media further comprise instructions to output the information to construct a short RNA molecule to a computer-readable medium.

The definitions and description above in relation to the methods of designing a short RNA molecule apply *mutatis mutandis* to these one or more computer-readable media.

Such media could be a physical (non-transitory) storage medium such as a ROM chip, CD ROM or disk, or could be a transitory medium or signal such as an electronic signal over wires, an optical signal or a radio signal.

It will further be appreciated that not all steps of the methods need be carried out by computer software. Thus the present disclosure includes computer software and such software installed on a computer software carrier for carrying out at least one of the steps of the methods set out herein.

The present disclosure accordingly includes a computer program product for use with an electronic device or system. Such an implementation may comprise a series of computer readable instructions either fixed on a tangible medium, such as a computer readable medium, for example, diskette, CD ROM, ROM, or hard disk, or transmittable to a computer system, via a modem or other interface device, over either a tangible medium, including but not limited to optical or analogue communications lines, or intangibly using wireless techniques, including but not limited to microwave, infrared or other transmission techniques. The series of computer readable instructions embodies all or part of the functionality previously described herein.

Those skilled in the art will appreciate that such computer readable instructions can be written in a number of programming languages for use with many computer architectures or operating systems. Further, such instructions may be stored using any memory technology, present or future, including but not limited to, semiconductor, magnetic, or optical, or transmitted using any communications technology, present or future, including but not limited to optical, infrared, or microwave. It is contemplated that such a computer program product may be distributed as a removable medium with accompanying printed or electronic documentation, for example, shrink wrapped software, pre loaded with a computer system, for example, on a system ROM or fixed disk, or distributed from a or the server or electronic bulletin board over a network, for example, the Internet or World Wide Web.

In a further aspect, the present disclosure includes a method of reprogramming a somatic or multipotent cell into a pluripotent cell by up-regulating a target gene in said cell, wherein said target gene is a pluripotency-inducing gene and wherein said method comprises contacting said cell with a short RNA molecule which specifically down-regulates a target RNA transcript present in said cell, wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is antisense to a genomic sequence located between 100 kb upstream of the target gene's transcription start site and 100 kb downstream of the target gene's transcription stop site.

The present disclosure also includes a method of maintaining or increasing the differentiation potential of a population of cells by up-regulating a target gene in said cells, wherein said target gene is a pluripotency-inducing gene and wherein said method comprises contacting said cells with a short RNA molecule which specifically down-regulates a target RNA transcript in said cells, wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is antisense to a genomic sequence located between 100 kb upstream of the target gene's transcription start site and 100 kb downstream of the target gene's transcription stop site.

A somatic cell is any type of cell forming the body of an organism with the exception of germ line cells (gametes), the cells from which gametes are made (gametocytes), multipotent cells and pluripotent cells. The somatic cell can be derived from any animal but is preferably a mammalian cell, most preferably a human cell.

A pluripotent cell is a cell that has the potential to differentiate into any of the three germ layers: endoderm (interior stomach lining, gastrointestinal tract, the lungs), mesoderm (muscle, bone, blood, urogenital), or ectoderm (epidermal tissues and nervous system). Differentiation potential is the extent to which a cell may differentiate into a cell of different types. A pluripotent cell has a greater differentiation potential than a multipotent cell. Within a population of cells, individual cells may possess different differentiation potentials. A population of multipotent cells may, after time, comprise some cells which have differentiated into somatic cells and some cells which have not differentiated and are still multipotent. Similarly, a population of pluripotent cells, after time, may contain multipotent cells and somatic cells as well as pluripotent cells. Thus, the above method of maintaining or increasing the differentiation potential of a population of cells may be used in connection with a population of somatic, multipotent or pluripotent cells.

An induced pluripotent stem cell (abbreviated as iPSC or iPS cell) is a type of pluripotent stem cell artificially derived from a non-pluripotent cell, typically an adult somatic cell, by inducing a "forced" expression of certain genes.

A multipotent cells is a cell which has the potential to give rise to cells from multiple, but a limited number of lineages. An example of a multipotent cell is a hematopoietic cell, a blood stem cell that can develop into several types of blood cells, but cannot develop into brain cells or other types of cells. Mesenchymal stem cells, or MSCs, are multipotent stem cells that into a variety of cell types including osteoblasts (bone cells), chondrocytes (cartilage cells) and adipocytes (fat cells).

Preferably all of the methods of the present disclosure are performed *in vitro.*

In the methods of the present disclosure, the reprogramming of somatic or multipotent cells into pluripotent cells or the induction of pluripotent stem cells is achieved by up-regulating i.e. activating a target pluripotency-inducing gene(s). The up-regulation is "cis" up-regulation. In this context "cis" up-regulation means that the target RNA transcript is transcribed from a locus which is associated with the locus of the target gene. Such "association" can be in one of three ways:
Firstly, the target RNA transcript can be transcribed from a locus up to 100 kb upstream of the target gene's transcription start site. Secondly, the target RNA transcript can be transcribed from a locus up to 100 kb downstream of the target gene's annotated transcription stop site. Thirdly, the target RNA transcript can be transcribed from a locus which interacts physically with the target gene. In this latter case, the target RNA transcript may be transcribed from a locus of any distance from the target gene's transcription start site or even on a different chromosome. It is well-known in the field that different regions of DNA are capable of long-range interactions either within the same chromosome or within different chromosomes [Lieberman-Aiden et al. (2009) Science 326: 289-293].

In contrast, "trans" up-regulation would occur if the target RNA transcript was not transcribed from a locus which was either within 100kb upstream of the target gene's transcription start site or within 100 kb downstream of the target gene's transcription stop site and was not transcribed from a locus which interacts physically with the target gene. In the methods of the present invention "trans" up-regulation is not contemplated.

Thus, the target RNA transcripts of the above methods are transcribed from a locus up to 100 kb upstream of the target gene's transcription start site, from a locus up to 100 kb downstream of the target gene's transcription stop site, or from a locus which interacts physically with the target gene. Preferably, the RNA transcripts are transcribed from a locus up to 60 kb upstream of the target gene's transcription start site, from a locus up to 60 kb downstream of the target gene's transcription stop site, or from a locus which interacts physically with the target gene. More preferably, the RNA transcripts are transcribed from a locus up to 40 kb upstream of the target gene's transcription start site, from a locus up to 40 kb downstream of the target gene's transcription stop site, or from a locus which interacts physically with the target gene. More preferably, the RNA transcripts are transcribed from a locus up to 20 kb upstream of the target gene's transcription start site, from a locus up to 20 kb downstream of the target gene's transcription stop site, or from a locus which interacts physically with the target gene. Optionally, the RNA transcripts are transcribed from a locus up to 1 kb upstream of the target gene's transcription start site, from a locus up to 1 kb downstream of the target gene's transcription stop site, or from a locus which interacts physically with the target gene. Optionally, the RNA transcripts are transcribed from a locus up to 100 nucleotides upstream of the target gene's transcription start site, from a locus up to 100 nucleotides downstream of the target gene's transcription stop site, or from a locus which interacts physically with the target gene.

The term "is transcribed from [a particular locus]" in the context of the target RNA transcripts of the invention means "the transcription start site of the target RNA transcript is found [at the particular locus]". The transcription start site of the target RNA transcript may be found on either strand of the chromosome containing the target gene, provided that the other essential features of the target RNA transcript are present. Preferably, the target RNA transcript of the present invention has its transcription start site and its transcription stop site within one of the regions i)a) or i)b) defined above. In other words, preferably both of the transcription start site and the transcription stop site of the target RNA transcript are, separately, located either up to 100 kb upstream of the target gene's transcription start site or up to 100 kb downstream of the target gene's transcription stop site. The preferred embodiments described above in relation to the location from which the target RNA transcript is transcribed apply *mutatis mutandis* to the location of the target RNA transcript's transcription stop site.

In the above methods, the target RNA transcript comprises a sequence which is antisense to a genomic sequence located between 100 kb upstream of the target gene's transcription start site and 100 kb downstream of the target gene's transcription stop site. More preferably, the target RNA transcript comprises a sequence which is antisense to a genomic sequence located between 60 kb upstream of the target gene's transcription start site and 60 kb downstream of the target gene's transcription stop site. More preferably, the target RNA transcript comprises a sequence which is antisense to a genomic sequence located between 40 kb upstream of the target gene's transcription start site and 40 kb downstream of the target gene's transcription stop site. More preferably, the target RNA transcript comprises a sequence which is antisense to a genomic sequence located between 20 kb upstream of the target gene's transcription start site and 20 kb downstream of the target gene's transcription stop site. More preferably, the target RNA transcript comprises a sequence which is antisense to a genomic sequence located between 1 kb upstream of the target gene's transcription start site and 1 kb downstream of the target gene's transcription stop site. More preferably, the target RNA transcript comprises a sequence which is antisense to a genomic sequence located between 100 nucleotides upstream of the target gene's transcription start site and ending 100 nucleotides downstream of the target gene's transcription stop site. Optionally the target RNA transcript comprises a sequence which is antisense to a genomic sequence which includes the coding region of the target gene.

The term "sense" when used to describe a nucleic acid sequence in the context of the present invention means that the sequence has identity to a sequence on the coding strand of the target gene. The term "antisense" when used to describe a nucleic acid sequence in the context of the present invention means that the sequence is complementary to a sequence on the coding strand of the target gene.

The terms "complementary" and "complementarity" are defined above. Preferably the target RNA transcript comprises a sequence which is at least 75%, preferably at least 85%, more preferably at least 90%, still more preferably at least 95% complementary along its full length to a sequence on the coding strand of the target gene. Preferably the target RNA transcript comprises a sequence which has perfect or near-perfect complementarity along its full length to a sequence on the coding strand of the target gene.

Alternatively, the target RNA transcript comprises one or more, usually several (e.g. at least 3 or at least 6), un-gapped sequences which have perfect or near-perfect complementarity to a sequence on the coding strand of the target gene, said un-gapped sequence being at least 16 nucleotides, more preferably at least 25 nucleotides, more preferably at least 50 nucleotides, still more preferably at least 75 nucleotides, most preferably at least 100 nucleotides in length.

In several aspects of the present invention the target RNA transcript may comprise a sequence which is sense to a sequence within the target gene, i.e. the target RNA transcript may comprise a sequence with identity to a sequence on the coding strand of the target gene. The terms "identity" and "identical" are defined above. Preferably the target RNA transcript comprises a sequence which is at least 75%, preferably at least 85%, more preferably at least 90%, still more preferably at least 95% identical along its full length to a sequence on the coding strand of the target gene. Preferably the target RNA transcript comprises a sequence which has perfect or near-perfect identity along its full length to a sequence on the coding strand of the target gene.

Alternatively, the target RNA transcript comprises one or more, usually several (e.g. at least 3 or at least 6), un-gapped sequences which have perfect or near-perfect identity to a sequence on the coding strand of the target gene, said ungapped sequence being at least 16 nucleotides, more preferably at least 25 nucleotides, more preferably at least 50 nucleotides, still more preferably at least 75 nucleotides, most preferably at least 100 nucleotides in length.

When assessing identity/complementarity between the RNA transcript(s) and the above-mentioned genomic sequence(s), the coding/template strands are considered to extend upstream and downstream of the gene's transcribed region, i.e. the terms "coding strand" and "template strand" are merely labels for the actual strands and do not indicate any length limitation.

The target RNA transcript is either a coding RNA molecule, i.e. an RNA molecule which codes for an amino acid sequence, or it is a non-coding RNA molecule, i.e. an RNA molecule which does not code for an amino acid sequence. Preferably the target RNA transcript is a non-coding RNA.

The target RNA transcripts are preferably at least 16 nucleotides in length. Preferably however the target RNA transcripts are at least 100, more preferably at least 200 nucleotides in length, most preferably at least 1000 nucleotides in length, possibly at least four thousand nucleotides in length.

In the above methods, the target RNA transcript comprises a sequence which "is antisense to a genomic sequence located between 100 kb upstream of the target gene's transcription start site and 100 kb downstream of the target gene's transcription stop site. For the sake of clarity, from hereon the term "genomic sequence" is used as a short hand for the term "genomic sequence located between 100 kb upstream of the target gene's transcription start site and 100 kb downstream of the target gene's transcription stop site". In other words, the target RNA transcript comprises a sequence which is complementary to a genomic sequence on the coding strand of the target gene.

Optionally, the genomic sequence to which the target RNA transcript is antisense comprises part of a promoter region of the target gene. In other words, optionally the target RNA transcript comprises a sequence which is antisense to a genomic sequence located between 100 kb upstream of the target gene's transcription start site and 100 kb downstream of the target gene's transcription stop site and which comprises part of a promoter region of the target gene. Another way of describing this feature is that the antisense target RNA transcript "overlaps" a promoter region of the target gene. Genes may possess a plurality of promoter regions, in which case the target RNA transcript may overlap with one, two or more of the promoter regions. Online database of annotated gene loci may be used to identify the promoter regions of genes.

For any given promoter region, the entire promoter region does not have to be overlapped, it is sufficient for a subsequence within the promoter region to be overlapped by the target RNA transcript, i.e. the overlap can be a partial overlap. Similarly, the entire target RNA transcript need not be antisense to the sequence within the promoter region, it is only necessary for the target RNA transcript to comprise a sequence which is antisense to the promoter region.

The region of overlap between the target RNA transcript and the promoter region of the target gene may be as short as a single nucleotide in length, although it is preferably at least 15 nucleotides in length, more preferably at least 25 nucleotides in length, more preferably at least 50 nucleotides in length, more preferably at least 75 nucleotides in length, most preferably at least 100 nucleotides in length. Each of the following specific arrangements are intended to fall within the scope of the term "overlap":
a) The target RNA transcript and the target gene's promoter region are identical in length and they overlap (i.e. they are complementary) over their entire lengths.
b) The target RNA transcript is shorter than the target gene's promoter region and overlaps over its entire length with the target gene's promoter region (i.e. it is complementary over its entire length to a sequence within the target gene's promoter region).
c) The target RNA transcript is longer than the target gene's promoter region and the target gene's promoter region is overlapped fully by it i.e. the target gene's promoter region is complementary over its entire length to a sequence within the target RNA transcript).
d) The target RNA transcript and the target gene's promoter region are of the same or different lengths and the region of overlap is shorter than both the length of the target RNA transcript and the length of the target gene's promoter region.

The above definition of "overlap" applies *mutatis mutandis* to the description of other overlapping sequences throughout the description. Clearly, if an antisense RNA transcript is described as overlapping with a region of the target gene other than the promoter region then the sequence of the transcript is complementary to a sequence within that region rather than within the promoter region. If referring to a sense target RNA transcript, the term "overlap" means that the target RNA transcript comprises a sequence which is sense to a sequence within the promoter region or other stated region of the target gene. In other words, the sense target RNA transcript comprises a sequence which is identical/has identity with a sequence on the coding strand of the target gene and within the specified region of the target gene.

Preferably the RNA transcript comprises a sequence which is antisense to a genomic sequence which comprises the target gene's transcription start site. In other words, preferably the target RNA transcript comprises a sequence which overlaps with the target gene's transcription start site.

Without wishing to be bound by theory, it is believed that the short RNAs of the present disclosure achieve modulation of the target gene by inducing the siRNA-like cleavage of the RNA transcript which is antisense (or, in some cases, sense) to a region of the target gene. Short RNAs of the present invention might also be able to act, in complex with Argonaute proteins, as anchors for regulatory chromatin-modifying proteins.

Methods of determining if an RNA transcript is present in a cell are well-known in the art. For instance, the genomic region around the locus of the gene of interest can be searched for spliced expresses sequence tags. An expressed sequence tag or EST is a short sub-sequence of a transcribed cDNA sequence. ESTs are commonly used to identify gene transcripts. Public databases of ESTs are known in the art, for instance the GenBank database. Alternatively, Reverse Transcriptase PCR (RT-PCR), a well-known tool for identifying RNA, can be used to identify potential target RNA transcripts. Alternatively, high throughput sequencing or other such methods can be used to sequence total, size-fractionated, or other suitable subsets of RNAs and use such sequencing libraries to identify RNA transcripts that originate from the region of interest. Alternatively, a population of known RNA transcripts can be searched to identify suitable transcripts. Any database of RNA transcripts known in the art can be used, for instance the University of California Santa Cruz (UCSC) Spliced EST track. Alternatively the population may be prepared from a population possessed by the skilled man working the methods herein for his own specific purposes. For instance, if the target gene is known to be expressed in a particular cell type, then the database of transcripts may be those which have been determined to be present in that cell type. The skilled man will be able to determine the population to use for his specific desired purposes.

In order to reprogram a somatic or multipotent cell into a pluripotent cell it is usually necessary for each of KLF4, POU5F1, SOX2 and MYC to be activated. The above-discussed methods require the use of short RNAs to up-regulate a target gene, i.e. at least one target pluripotency-inducing gene. The methods therefore permit those pluripotency-inducing genes not activated by the use of the short RNAs of the invention to be activated by other means known in the art. Optionally, the above methods comprise the up-regulation of 2 or 3 target genes selected from the group consisting of KLF4, POU5F1, SOX2 and MYC by using the short RNAs of the invention. Preferably, at least one of the target genes up-regulated by the short RNAs of the present invention is KLF4. Optionally the methods comprise the up-regulation of each of KLF4, POU5F1, SOX2 and MYC by the short RNAs of the invention. Optionally, such methods further comprise the up-regulation of NANOG or/and LIN28 by any method known in the art. Preferably, if the methods comprise the step of up-regulating NANOG or/and LIN28 the up-regulation is achieved by the use of the short RNA molecules of the disclosed herein.

In the above method the cell or population of cells is contacted with a short RNA molecule of the present disclosure. The short RNA molecules can be administered to said cells by using any suitable delivery reagents in conjunction with the present short RNAs. Such suitable delivery reagents include the Mirus Transit TKO lipophilic reagent; lipofectin; lipofectamine; cellfectin; or polycations (e.g., polylysine), virus-based particles, electroporation or liposomes. A preferred delivery reagent is a liposome. A variety of methods are known for preparing liposomes, for example as described in Szoka et al. (1980), Ann. Rev. Biophys. Bioeng. 9: 467; and U.S. Pat. Nos. 4,235,871 and 5,019,369.

Particularly preferably, the liposomes encapsulating the present short RNAs are modified so as to avoid clearance by the mononuclear macrophage and reticuloendothelial systems, for example by having opsonization-inhibition moieties bound to the surface of the structure. In one embodiment, a liposome can comprise both opsonization-inhibition moieties and a ligand.

Recombinant plasmids which express the short RNAs can also be administered directly or in conjunction with a suitable delivery reagent, including the Mirus Transit LT1 lipophilic reagent; lipofectin; lipofectamine; cellfectin; polycations (e.g., polylysine) or liposomes. Recombinant viral vectors which express the short RNA and methods for delivering such vectors to a cell are known within the art.

Preferably said contacting step is performed daily or every alternate day for at least one day, preferably at least four days, more preferably at least 6 days, still more preferably at least 8 days, still more preferably at least 12 days, still more preferably about 18 to 23 days, most preferably about 21 days. Preferably said contacting step is performed once, twice or thrice daily or every alternate day. In the above methods, if more than one target gene is up-regulated then the short RNAs used to up-regulate the different target genes may be administered at different frequencies and for different lengths of time. The particular administration regimens to be used can be readily determined by one of ordinary skill in the art to suit his desired purpose, particular starting cell type and delivery method. By way of example, picoMolar concentrations of the short RNA molecules of the present disclosure may be used. The short RNA of the disclosure herein may be provided alone or in combination other active agent(s) known to have an effect in the particular method being considered. The other active agent(s) may be administered simultaneously, separately or sequentially with the short RNA. Thus, it is possible to use a single short RNA, combination of two or more short RNAs
or, if applicable, a combination of said short RNA(s) and other active substance(s).

In a further aspect the present disclosure includes a method of up-regulating a target gene, wherein said target gene is a pluripotency-inducing gene and wherein said method comprises contacting a cell comprising said target gene with a short RNA molecule which specifically down-regulates a target RNA transcript present in said cell, wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is antisense to a genomic sequence located between 100 kb upstream of the target gene's transcription start site and 100 kb downstream of the target gene's transcription stop site.

The definitions and description above in relation to the methods of reprogramming a somatic or multipotent cell into a pluripotent cell or maintaining or increasing the differentiation potential of a population of cells apply *mutatis mutandis* to this method of up-regulating a target pluripotency-inducing gene.

In a further aspect the present disclosure includes a method of down-regulating a target gene, wherein said target gene causes differentiation and wherein said method comprises contacting a cell comprising said target gene with a short RNA molecule which specifically down-regulates a target RNA transcript present in said cell, wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is sense to a genomic sequence located between 100 kb upstream of the target gene's transcription start site and 100 kb downstream of the target gene's transcription stop site.

The above method can be used in isolation or, if desired, as an additional step in the above-discussed methods of reprogramming a somatic or multipotent cell into a pluripotent cell or maintaining or increasing the differentiation potential of a population of cells. The inhibition of differentiation may be advantageous in the preparation of pluripotent cells so that they do not proceed to differentiate uncontrollably.

Unless otherwise stated, the definitions and description above in relation to the methods of reprogramming a somatic or multipotent cell into a pluripotent cell or maintaining or increasing the differentiation potential of a population of cells apply *mutatis mutandis* to this method of down-regulating a target gene which causes differentiation.

In this method of down-regulating a target gene which causes differentiation, the down-regulation is "cis" down-regulation. The term "cis" in this context is as described above.

In this method of down-regulating a target gene which causes differentiation, the target RNA transcript is sense to the target gene. The term "sense" is as described above.

In this method of down-regulating a target gene which causes differentiation, the target RNA transcript comprises a sequence which is sense to a genomic sequence located between 100 kb upstream of the target gene's transcription start site and 100 kb downstream of the target gene's transcription stop site. In other words, the target RNA transcript comprises a sequence which is identical/has identity to a sequence on the coding strand of the target gene located between 100 kb upstream of the target gene's transcription start site and 100 kb downstream of the target gene's transcription stop site.

Optionally, the genomic sequence to which the target RNA transcript is sense comprises part of a promoter region of the target gene. In other words, optionally the target RNA transcript comprises a sequence which is sense to a genomic sequence located between 100 kb upstream of the target gene's transcription start site and 100 kb downstream of the target gene's transcription stop site and which comprises part of a promoter region of the target gene. Another way of describing this feature is that the sense target RNA transcript "overlaps" a promoter region of the target gene. Genes may possess a plurality of promoter regions, in which case the target RNA transcript may overlap with one, two or more of the promoter regions. Online database of annotated gene loci may be used to identify the promoter regions of genes.

For any given promoter region, the entire promoter region does not have to be overlapped, it is sufficient for a subsequence within the promoter region to overlapped by the target RNA transcript i.e. the overlap can be a partial overlap. Similarly, the entire target RNA transcript need not be sense to the sequence within the promoter region, it is only necessary for the target RNA transcript to comprise a sequence which is sense to the promoter region. The regions of overlap are as defined above.

Preferably the RNA transcript comprises a sequence which is sense to a genomic sequence which comprises the target gene's transcription start site. In other words, preferably the target RNA transcript comprises a sequence which overlaps with the target gene's transcription start site.

In a further aspect, the present disclosure includes an algorithm for the design of a short RNA molecule which modulates the expression of a target gene in a cell, said algorithm comprising the following steps:
(i) identify a population of potential target RNA transcripts present in said cell which are transcribed from:
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene;
(ii) if up-regulation of said target gene is desired, identify those RNA transcripts identified in step (i) which are antisense to the target gene, or, if down-regulation of said target gene is desired, identify those RNA transcripts identified in step (i) which are sense to the target gene;
(iii) from the RNA transcripts identified in step (ii), identify those RNA transcripts which comprise a sequence which overlaps with a genomic sequence located between 100 kb upstream of the target gene's transcription start site and 100 kb downstream of the target gene's transcription stop site; and
(iv) generate a short RNA sequence which is complementary to the sense or antisense non-coding RNA transcript identified in step (iii).

A key feature of the above algorithm, and indeed to all aspects of the present disclosure is that targeting antisense RNA transcripts with the short RNAs of the present invention leads to up-regulation of the target gene while targeting sense RNA transcripts leads to down-regulation of the target gene.

The identification of potential RNA transcripts in step (i) can be performed by any method known in the art. For instance, the identification of potential antisense transcripts can be performed by searching the genomic region around the locus of the gene of interest for spliced expresses sequence tags. An expressed sequence tag or EST is a short sub-sequence of a transcribed cDNA sequence. ESTs are commonly used to identify gene transcripts. Public databases of ESTs are known in the art, for instance the GenBank database.

Such databases typically disclose not only the position of the EST in terms of its distance from the target gene's transcription site, but also in terms of the strand on which it is located and the direction and length of its transcription. Thus, any of steps (i), (ii) and (iii) may be performed as a combined step in which target RNA transcripts which satisfy all of the requirements recited in steps (i) to (iii) above are identified in one search step. For instance, the database of ESTs can be searched for ESTs which
i) are located
   a) up to 100 kb upstream of the target gene's transcription start site,
   b) up to 100 kb downstream of the target gene's transcription stop site; or
   c) a locus which interacts physically with the target gene;
ii) are present either on the target gene's coding strand (if the identification of sense transcripts is desired) or on the target gene's template strand (if the identification of antisense transcripts is desired); and
iii) mark the site of the initiation of transcription of an RNA molecule which is sufficient in length and transcribed in the required direction to overlap a genomic sequence located between 100 kb upstream of the target gene's transcription start site and 100 kb downstream of the target gene's transcription stop site.
   Unless otherwise stated, the definitions and description above in relation to the methods of the present invention apply *mutatis mutandis* to the algorithm aspect of the present disclosure.
   Steps (i), (ii) and (iii) of the above algorithm may be performed in any order. Steps (i) to (iii) must however be performed before step (iv).
   Alternatively, Reverse Transcriptase PCR (RT-PCR), a well-known tool for identifying RNA, can be used to identify potential target RNA transcripts. Alternatively, high throughput sequencing or other such methods can be used to sequence total, size-fractionated, or other suitable subsets of RNAs and use such sequencing libraries to identify RNA transcripts that originate from the region of interest.
   Alternatively, a population of known RNA transcripts can be searched to identify those which satisfy the criteria above. Any database of RNA transcripts known in the art can be used, for instance the University of California Santa Cruz (UCSC) Spliced EST track. Alternatively the population may be prepared from a population possessed by the skilled man working the methods herein for his own specific purposes. For instance, if the target gene is known to be expressed in a particular cell type, then the database of transcripts may be those which have been determined to be present in that cell type. The skilled man will be able to determine the population to use for his specific desired purposes.
   Step (iv) of the above algorithm requires the design of a short RNA molecule which gives effective and specific down-regulation of the sense or antisense non-coding RNA transcript identified in step (iii). The short RNA molecule may be designed to be as defined anywhere above. The above algorithm may thus comprise further steps, or modified versions of the steps above, which require the design or selection of a short RNA molecule with the properties described anywhere above. The discussion above details the tools and methods well-known to those skilled in the art which can be used to perform these steps. Preferably the above algorithm comprises the following step (iv):
(iv) generate a short RNA molecule which is complementary to the sense or antisense non-coding RNA transcript identified in step (iii) and which, through hybridisation after administration to a cell comprising the sense or antisense non-coding RNA transcript identified in step (iii), would achieve down-regulation of the sense or antisense non-coding RNA transcript identified in step (iii).

The target RNA transcripts identified in the above algorithm may be as defined anywhere above. Therefore, the above algorithm may possess further steps or modified versions of the above-discussed steps which require the identification of target RNA transcripts with properties as defined anywhere above.

In particular, preferably the above algorithm comprises a further step (iii)(a) performed prior to step (iv):
(iii)(a) from the RNA transcripts identified in step (iii), identify those which comprise a sequence which is antisense to a genomic sequence which comprises part of a promoter region of the target gene or the target gene's transcription start site.

Steps (i), (ii), (iii) and (iii)(a) may be performed in any order provided they are performed before step (iv).

Alternatively, the above algorithm may comprise a further step (iii)(a)' performed prior to step (iv):
(iii)(a)' from the RNA transcripts identified in step (iii), identify those which comprise a sequence which is sense to a genomic sequence which comprises part of a promoter region of the target gene or the target gene's transcription start site.

Steps (i), (ii), (iii) and (iii)(a) may be performed in any order provided they are performed before step (iv).

In the above algorithm the target gene may be any of the target genes described above. Preferably the target gene is a pluripotency inducing gene or a gene which causes differentiation, more preferably a pluripotency-inducing gene. Still more preferably the pluripotency-inducing gene is selected from the group consisting of KLF4, POU5F1 (also called OCT3/4), SOX2, MYC, NANOG and LIN28, more preferably selected from the group consisting of KLF4, POU5F1 (also called OCT3/4), SOX2 and MYC. Most preferably the target gene is KLF4.

In a further aspect the present disclosure includes a method of designing a short RNA molecule which comprises performing an algorithm as defined above.

In a further aspect the present disclosure includes a method of producing a short RNA molecule which comprises performing an algorithm as defined above and then synthesizing one or more of the RNA molecules generated by said algorithm.

In a further aspect the present disclosure includes a short RNA molecule which specifically up-regulates a target gene in a cell by down-regulating a target RNA transcript present in said cell,wherein said target gene is a pluripotency-inducing gene and wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is antisense to a genomic sequence located between 100 kb upstream of the target gene's transcription start site and 100 kb downstream of the target gene's transcription stop site.

In a further aspect the present disclosure includes a short RNA molecule which specifically down-regulates a target gene in a cell by down-regulating a target RNA transcript present in said cell, wherein said target gene is a gene which causes differentiation and wherein said target RNA transcript:
i) is transcribed from
   a) either strand of a locus up to 100 kb upstream of the target gene's transcription start site,
   b) either strand of a locus up to 100 kb downstream of the target gene's transcription stop site; or
   c) either strand of a locus which interacts physically with the target gene; and
ii) comprises a sequence which is sense to a genomic sequence located between 100 kb upstream of the target gene's transcription start site and 100 kb downstream of the target gene's transcription stop site.

Unless otherwise stated, the definitions and description above in relation to the methods of the present disclosure apply *mutatis mutandis* to the product aspects of the present disclosure.

As discussed in the Examples, using the above algorithm, the present inventors have designed specific short RNA molecules which effectively modulate the activity of numerous genes. Thus, in a further aspect the present disclosure includes short RNA molecules with the specific sequences shown in the Tables below.

**Table 1**

| Activating small RNA (saRNA) candidates against KLF4. | | | | | |
|---|---|---|---|---|---|
| The table lists the two most promising siRNAs against the antisense EST DB461753 (IDs DB-1 and DB-2) and KLF4's promoter region (IDs Pr-1 and Pr-2). "Pos" is the target site start within the EST or the KLF4 promoter region; "Exon" is the target site's exon number; "Sense" shows the siRNAs' 19mer target site sequence; and "Antisense" shows the corresponding reverse-complementary sequence. The sense and antisense sequences plus 2 nt overhang sequences at their 3' ends (UU; not listed in the table) form the siRNA duplex candidates. | | | | | |
| ID | Target | Pos | Exon | Sense (passenger) | Antisense (guide) |
| DB-1 | DB461753 | 416 | 2 | GACCAUAUUUCUCUUGAAU | AUUCAAGAGAAAUAUGGUC |
| DB-2 | DB461753 | 313 | 2 | ACAAGGCUUCCAUUAAAGA | UCUUUAAUGGAAGCCUUGU |
| Pr-1 | AS TSS+/-500 | 514 | n/a | GCGCGUUCCUUACUUAUAA | UUAUAAGUAAGGAACGCGC |
| Pr-2 | AS TSS+/-500 | 26 | n/a | CUUCUUUGGAUUAAAUAUA | UAUAUUUAAUCCAAAGAAG |

**Table 2**

| Activating small RNA (saRNA) candidates against MYC. POU5F1. and SOX2. | | | | | | |
|---|---|---|---|---|---|---|
| The table lists the two most promising siRNAs against antisense ESTs and promoter regions of MYC, POU5F1, and SOX2. | | | | | | |
| Gene | ID | Target | Pos | Exon | Sense (passenger) | Antisense (guide) |
| MYC | BC-1 | BC042052 | 63 | 1 | GUGACUAUUCAACCGCAUA | UAUGCGGUUGAAUAGUCAC |
| MYC | BC-2 | BC042052 | 31 | 1 | GAGGAGUUACUGGAGGAAA | UUUCCUCCAGUAACUCCUC |
| MYC | Pr-1 | AS TSS+/- 500 | 787 | n/a | AGCAGUACUGUUUGACAAA | UUUGUCAAACAGUACUGCU |
| MYC | Pr-2 | AS TSS+/- 500 | 322 | n/a | GAAUUACUACAGCGAGUUA | UAACUCGCUGUAGUAAUUC |
| POU5F1 | BG-1 | BG203640 | 664 | 3 | UUUAAAUUCAAGAGAUCUA | UAGAUCUCUUGAAUUUAAA |
| POU5F1 | BG-2 | BG203640 | 622 | 2 | CGAGAACACCUGUCAAGUU | AACUUGACAGGUGUUCUCG |
| POU5F1 | Pr-1 | AS TSS+/- 500 | 940 | n/a | AUUCCUGUCCUCAAGAAAU | AUUUCUUGAGGACAGGAAU |
| POU5F1 | Pr-2 | AS TSS+/- 500 | 479 | n/a | UGAAAUGAGGGCUUGCGAA | UUCGCAAGCCCUCAUUUCA |
| SOX2 | BG-1 | BG220229 | 338 | 3 | AAAGGUCAUCUGACAUAAU | AUUAUGUCAGAUGACCUUU |
| SOX2 | BG-2 | BG220229 | 6 | 1 | CUGCUUUCCACCUAUGAAA | UUUCAUAGGUGGAAAGCAG |
| SOX2 | Pr-1 | AS TSS+/- 500 | 519 | n/a | GGGCUGUCAGGGAAUAAAU | AUUUAUUCCCUGACAGCCC |
| SOX2 | Pr-2 | AS TSS+/- 500 | 464 | n/a | UGACAACUCCUGAUACUUU | AAAGUAUCAGGAGUUGUCA |

**Table 3**

| Activating small RNA (saRNA) candidates against BCL2 and IL8. | | | |
|---|---|---|---|
| Gene | ID | Sense (passenger) | Antisense (guide) |
| BCL2 | PR1 | GAGGAUUUCCAGAUCGAUUUU | AAUCGAUCUGGAAAUCCUCUU |
| BCL2 | PR2 | UCAGCACUCUCCAGUUAUAUU | UAUAACUGGAGAGUGCUGAUU |
| BCL2 | PR3 | GCAGGAAUCCUCUUCUGAUUU | AUCAGAAGAGGAUUCCUGCUU |
| BCL2 | PR4 | GCAGAAGUCCUGUGAUGUUUU | AACAUCACAGGACUUCUGCUU |
| IL8 | PR1 | UUCAUUAUGUCAGAGGAAAUU | UUUCCUCUGACAUAAUGAAUU |
| IL8 | PR2 | CGCUGUAGGUCAGAAAGAUUU | AUCUUUCUGACCUACAGCGUU |

The disclosure also includes single-stranded RNA molecules comprising or consisting of the above individual strand sequences.

The disclosure also includes DNA molecules equivalent to the above mentioned RNA molecules.

In a further aspect the present disclosure includes a cell comprising a short RNA described herein. In a further aspect the present disclosure includes a pluripotent cell prepared by any one of the methods of the present disclosure and uses of such cells in therapy.

In a further aspect the present disclosure includes a short RNA for use in therapy.

In a further aspect, the disclosure includes a method of gene therapy comprising administering to a patient in need thereof a short RNA. The present disclosure includes a short RNA for use in the treatment of a disease associated with a deficiency of pluripotent cells or multipotent cells in a patient.

Optionally, the present disclosure includes a short RNA for use in the regeneration of the haematopoietic system of a patient deficient in pluripotent or mulipotent cells.

The short RNA molecules disclosed herein may be used directly in therapeutic methods, including methods of regeneration or repair. Optionally the regeneration or repair is of damaged organs. Alternatively, the regeneration or repair may be of an organ which has not been 'damaged' as such but which has not developed in the normal way. 'Regeneration' should thus be interpreted broadly to include all methods of organ growth or improvement.

The short RNAs disclosed herein may be administered to a patient in need thereof by any means or delivery vehicle known in the art, for example via nanoparticles, cationic lipids, polymers, dendrimers, aptamers, or as antibody siRNA conjugates, viral vector expressed shRNAs or miRNA mimics.

Various documents including, for example, publications and patents, are recited throughout this disclosure.

The citation of any given document is not to be construed as an admission that it is prior art with respect to the present invention. To the extent that any meaning or definition of a term in this written document conflicts with any meaning or definition of the term in a document cited herein, the meaning or definition assigned to the term in this written document shall govern.
Figure 1 is a schematic diagram showing the KLF4 locus and potential antisense target candidates. The Figure shows the genomic location of KLF4, the structure of the KLF4 transcript, and spliced ESTs from the surrounding regions (image adapted from the UCSC genome browser). Red boxes outline the KLF4 promoter region and the closest antisense EST upstream of KLF4 (DB461753). The antisense EST DB461753 initiates roughly 15 kb from KLF4's transcription start site (TSS) and terminates more than 25 kb away. Red arrows indicate potential target sites for small RNA candidates.

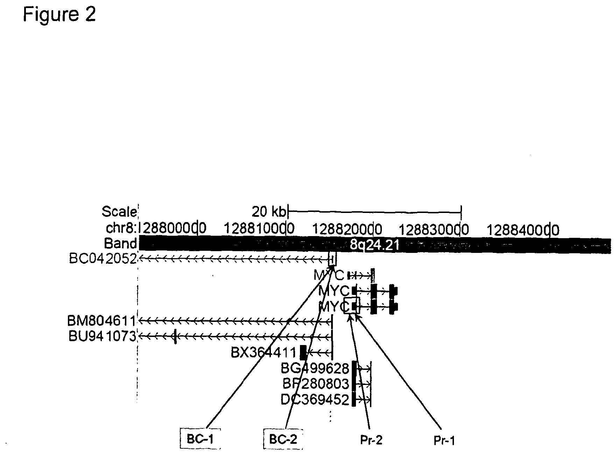

Figure 2 is a schematic diagram showing the MYC locus and potential antisense target candidates. The figure shows the genomic location of MYC, the structure of the MYC transcript, and spliced ESTs from the surrounding regions (image from the UCSC genome browser). Red boxes outline the MYC promoter region and the closest antisense transcript upstream of MYC (BC042052). The antisense ncRNA gene BC042052 is located about 2000 nts upstream of MYC. Red arrows indicate potential target sites for small RNA candidates.

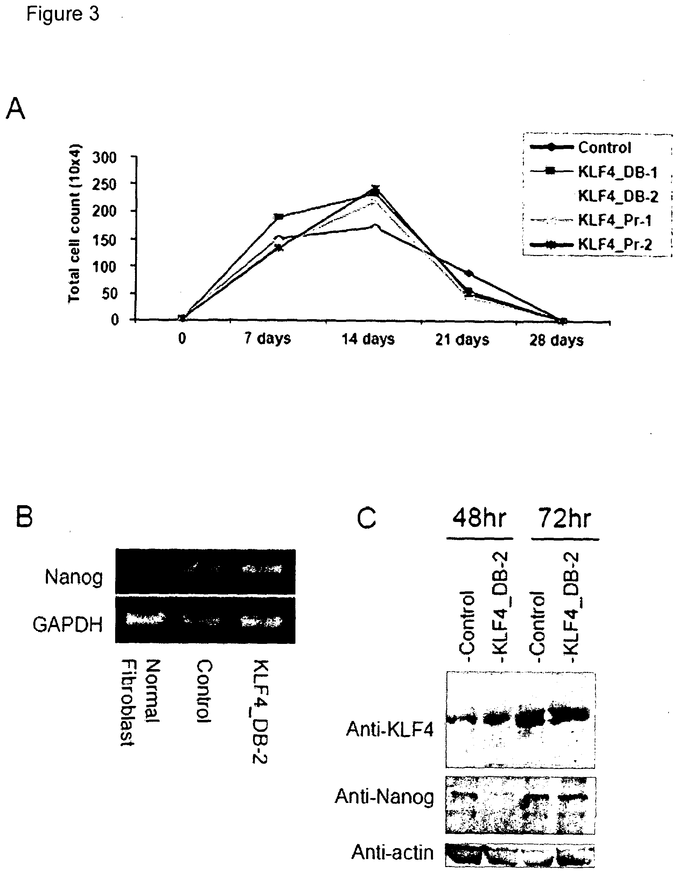

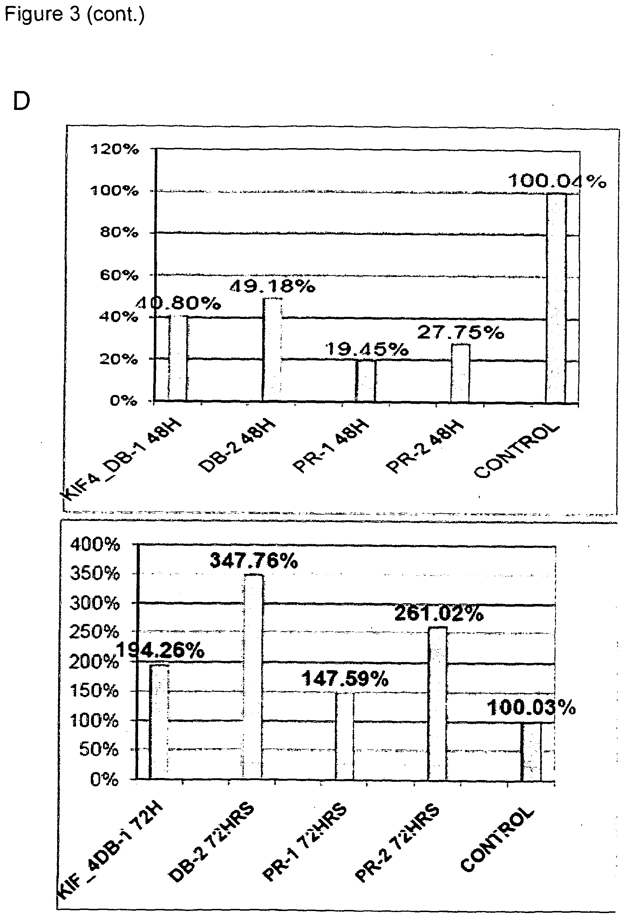

Figure 3 demonstrates that KLF4 short activating RNAs give rapid and increased expansion of Cd34+ cells (OmniCytes). (A) Cd34+ cells were treated with KLF4 short activating RNAs (saRNAs) or a control and cell growth were monitored for 28 days. Three of the four saRNAs gave increased cell counts compared with the control-treated cells, with the DB-1 saRNA resulting in the most rapid cell expansion. (b-c) Nanog expression levels in DB-2 treated cells 72 h post transfection as measured by (B) RT-PCR or (C) immunoblot. (D) saRNAs induce KLF4 expression. KLF4 expression was measured by RT-PCR in cells treated by saRNAs or a control 48 h and 72 h post treatment (top and bottom). After 72 h, all saRNAs gave increased KLF4 expression relative to the control, with DB-2 resulting highest KLF4 expression.
Figure 4 shows the results of qRT-PCR of KIf4 treated cells showing increase in (A) KIf4 and (B) Sox2 expression following KIf4 siRNA treatment in CD34+ cells, relative to control-treated cells.
Figure 5 shows Myc-expression in MSCs treated with c-Myc activating oligos, relative to control-treated cells.
Figure 6 shows KLF4-expression in MSCs treated with KLF4 activating oligo candidates, relative to control-treated cells. In this case, the oligos were added to the medium of Mesenchymal Stem Cells every day for 8 days. The activation effect is more prolonged for the functional oligo PR-1 than for the other oligos and confirms that KLF4-PR1 up-regulates KLF4 in MSCs.
Figure 7 shows the effect of Klf-activating oligo candidates on KIf4 expression in MSCs relative to control-treated cells.
Figure 8 shows the RT-qPCR result for Nanog when MSCs were exposed to the successful KIf4 activating oligo, Klf4-PR1, relative to control-treated cells. This shows that the activation of KIf4 affects transcription of downstream genes. Myc was up-regulated by 2.5-fold (Figure 7), and Nanog was up-regulated by 300-fold.

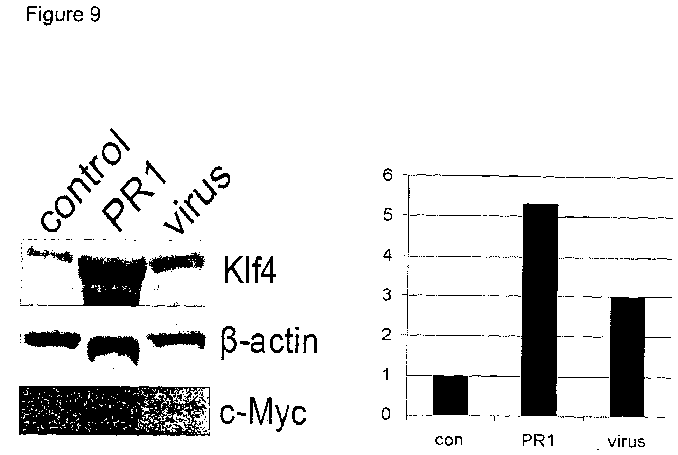

Figure 9 shows Western blot confirmation of KLF4 up-regulation at the protein level in hMSCs after treatment with KLF4-targeted PR1 saRNA oligo. The left panels show a Western blot probed with antibodies against KLF4 (upper left panel), beta-actin (middle left panel, to confirm equal loading in each lane), or c-Myc. Lanes: Control = Negative control MSCs treated with scrambled sequence control RNA oligo, PR1: MSCs treated with PR1 saRNA oligo, Virus: Positive control MSCs treated with lentivirus vector expressing exogenous KLF4 transgene, driven by CMV promoter. A clear up-regulation of KLF4, as well as c-MYC, at the protein level, can be seen in the PR1 lane, with a smaller increase in KLF4 and c-MYC levels seen in the Virus (positive control) lane. The right panel shows luminometric quantitation of the Western blot band intensities. The Y axis represents the relative band intensity in terms of fold increase over the Control (scrambled sequence oligo, set as 1).

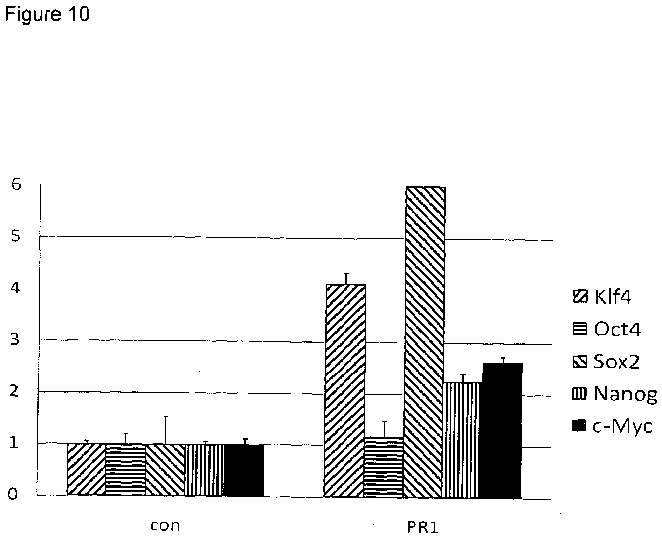

Figure 10 shows RT-qPCR results for KIf4, Oct4, Sox2, Nanog, and c-Myc mRNA expression levels on Day 8 after MSCs were exposed to the successful KIf4 activating oligo, KIf4-PR1. The results show that KIf4 activation by PR1 oligo also causes activation of Sox2, Nanog, and Myc.

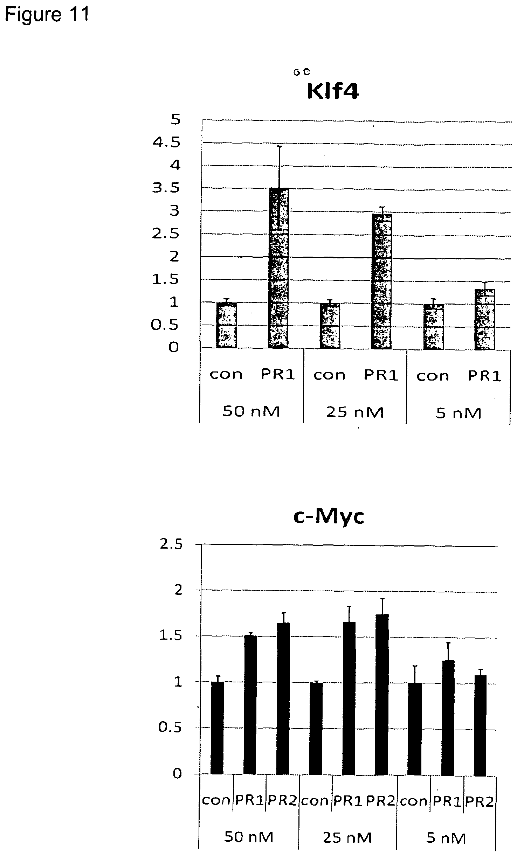

Figure 11 shows RT-qPCR results showing the effect of different doses of the Klf activating oligo candidates on KIf4 expression in MSCs, and the c-Myc activating oligo on c-Myc expression in MSCs on Day 8. The oligo doses tested (5 nM, 25 nM, 50 nM) are as indicated in each graph.

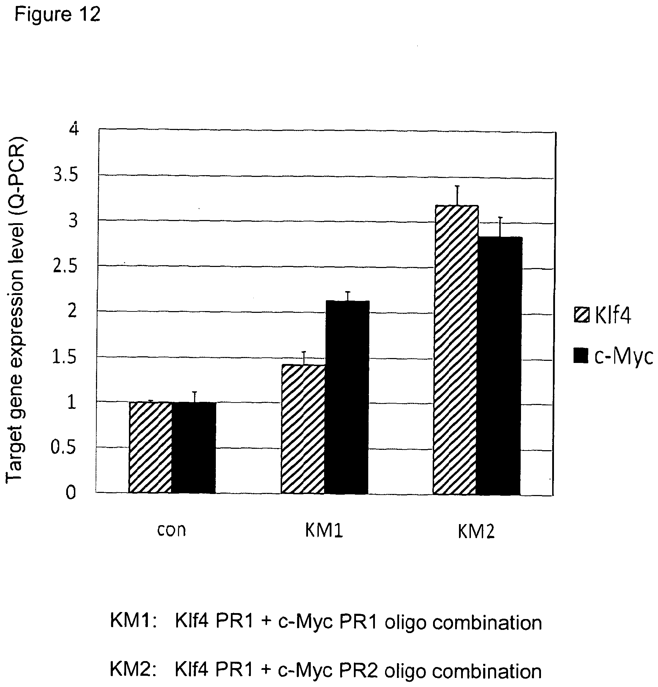

Figure 12 shows RT-qPCR results showing the effect in MSCs on Day 8, after treatment with KIf4 oligo PR1 combined with c-Myc oligo PR1 or PR2. c-Myc activation appears to be higher when combined with KIf4 oligo than with c-Myc oligo alone.
Figure 13 shows A) RT-qPCT results showing the effect in HepG2 cells of transfection with the BCL2-targeting saRNAs PR1, PR2, PR3 and PR4; B) RT-qPCT results showing the effect in Omnicytes of transfection with the BCL2-targeting saRNAs PR1, PR2, PR3 and PR4; C) RT-qPCT results showing the effect in HepG2 cells of transfection with the IL8-targeting saRNAs PR1, PR2 and PR3; D) RT-qPCT results showing the effect in Omnicytes of transfection with the IL8-targeting saRNA PR1.

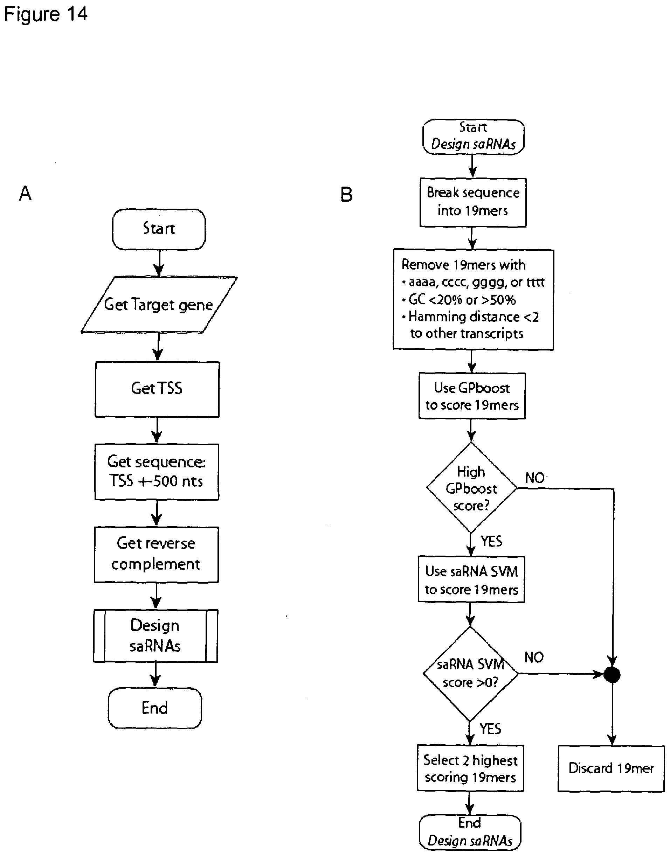

Figure 14 shows A) a flow diagram outlining the steps a) to c) of the design method of the present invention; B) A flow diagram outlining optional additional steps of steps c) and ii) of the design method of the present disclosure.

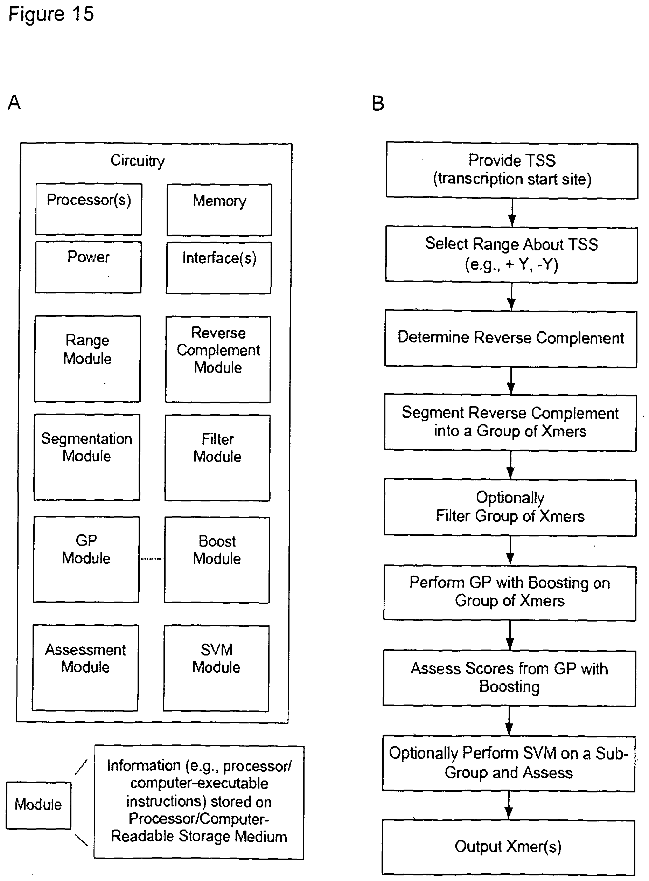

Figure 15 shows A) the circuitry arrangements within a computer implementation of the design method of the present invention; B) A flow diagram outlining a preferred design method of the present disclosure.

### Examples

### Example 1

### Summary

The aim of the study was to ascertain whether the expression of the pluripotency genes such as KIf4, Myc, Sox2 and Nanog could be up-regulated using a non-genetic approach by the addition of short RNAs. Synthetic oligos were designed to up-regulate KIf4 [the master regulator that controls the expression of other pluripotency factors] and Myc proteins and tested their effects on CD34+ haematopoietic stem cells and mesenchymal stem cells.

Four constructs were designed; DB1 and DB2 that targets the antisense in the EST region and PR1 and PR2 that targets an antisense sequence in the promoter region of the KIf4 gene.

In Haematopoietic CD34+ cells, KIf4-activating oligos led to increase KIf4 expression with DB1 and DB2 constructs. This was associated with increased cell proliferation. Another construct, the KIf4-PR2 construct, led to increased expression of the Sox2 gene product. In mesenchymal stem cells; the Myc activating oligos PR1 and PR2 led to up-regulation of c-myc. Like wise the KIf4-PR1 activating oligo led to up-regulation of KIf4 protein as well as KIf4-regulated genes c-myc and nanog.

In conclusion single and double-stranded oligos can lead to up-regulation of the pluripotency genes in adult bone marrow derived stem cells and this may have practical applications

### Materials and Methods

### Cell growth curve

Hematopoietic CD34⁺ stem cells derived from the bone marrow were cultured according to Gordon et al., (2006) Stem Cells 24(7): 1822-30. Briefly, bone marrow derived CD34⁺ cells were isolated from mononuclear cells using the CD34⁺ isolation kit (Miltenyi Biotechnology). For the growth curve analysis, cells (1x10⁵) were transfected using the Nanofectamine reagent according to the manufactures protocol (PAA Ltd) with individual KLF4 oligonucleotides (100nM). The KLF4 oligonucleotides tested were KLF4_DB-1, KLF4_DB-2, KLF4_Pr-1, and KLF4_Pr-1. Cells were transfected every 7 days during the 28 days of expansion period. Total live cells were counted once a week and replaced with fresh medium.

For Mesenchymal stem cells [MSCs] In all experiments, 20,000 MSCs (Passage P8 for the KIf4 tests, Passage P5 for c-Myc) were seeded in replicate wells on Day - 1, and each well was transfected with 50nM of a candidate oligo using Lipofectamine RNAiMAX on Days 0, 2, 4, and 6. Cells were lysed and RNA isolated with the QIAGEN RNeasy kit on Days 2, 4, 6, and 8. Reverse transcription to generate cDNA was done with the ABI High Capacity cDNA Kit, and the samples were amplified by qPCR with ABI Taqman Gene Expression Master Mix, all according to the manufacturers' standard protocols. Beta-actin was used as an internal control and samples were normalized to the scrambled sequence control oligo by the relative quantitation method. Nanog activation by the KIf4 oligo is shown on a log scale because the RQ was >300x.

### Western blotting

For the Western blot analysis, KLF4_DB-2 oligonucleotide was transfected into 1x10⁵ cells using the Nanofectamine reagent following the manufacturer's recommendation (PAA). Total protein lysates were collected at 48 hours and 72 hours post-transfection in a lysis buffer (1% NP-40 and 1% Triton-X100 in PBS). The 72 hours harvested RNA received two sequential transfection of the KLF4_DB-2 oligonucleotide. The protein concentration was measured using the protein DC assay (Bio-rad). Approximately 100ug of protein was loaded and resolved using standard SDS-PAGE on to Novex 4-20% Tris-Glycine Gels (Invitrogen). Proteins were separated by gel electrophoresis and transferred onto nitrocellulose membrane using a semi-dry blotting apparatus (Bio-Rad). The membranes were blocked in TBS containing 5% non-fat milk for 1 hour before incubating with primary antibodies for 1 hour at room temperature. The primary antibody against KLF4 (Millipore) at 1:200 dilution, Nanog (R&D systems) at 1:200 dilution, actin (Sigma) at 1:500 dilution were used to probe the blot followed by appropriate secondary conjugated alkaline-phosphatase (Jackson Laboratory) at 1:5000 dilution. After several washes, the blots were detected using BCIP/NTB reagent (Calbiochem). The blots were imaged using Geldoc system (UVP).

### RT-PCR

The KLF4_DB-2 oligonucleotide was transfected into 1x10⁵ cells. Total RNA was harvested post-transfection at 48 hours and 72 hours. The RNA isolated at 72 hours received two sequential transfection of oligonucleotide.
Total RNA was recovered using the RNAqueous-Micro kit (Ambion) following the manufacturer's recommendation. The RNA was quantified using a Nanodrop 2000 micro-sample quantitator. Approximately 200ng of total RNA from each sample was reverse transcribed using the One Step RT-PCR kit (Qiagen) following the manufacturer's recommendation. Expression of human Nanog was measured semi-quantitatively by PCR using a primer pair (R&D systems) under 32 cycles at 94°C for 45sec, 55°C for 45 sec and 72°C for 45 sec. GAPDH primers: Forward (5' GTGAAGGTCGGAGTCAACG3') and Reverse
(5'GGTGAAGACGCCAGTGGACTC3') was used as a loading control under 36 cycles at 94°C for 45sec, 60°C for 45 sec and 72°C for one minute. The PCR product was analysed on an agarose gel and imaged using a Geldoc system (UVP).

### Results

### Designing short RNAs for activating KLF4 expression

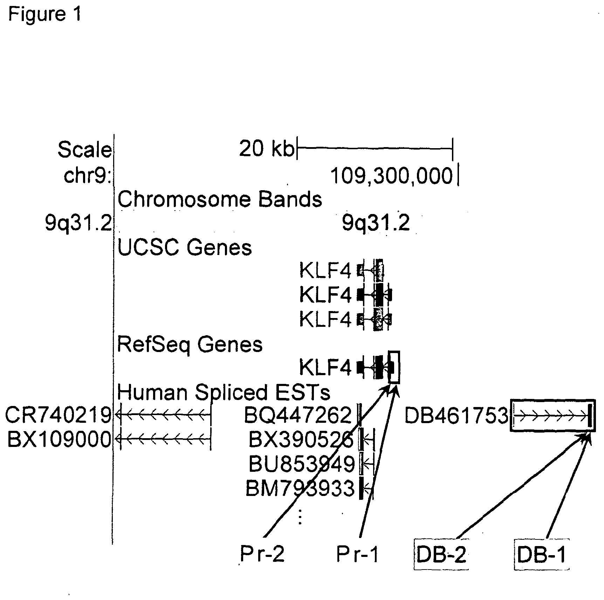

KLF4 is located in band 31, sub-band 2 of the long arm of chromosome 9 (9q31.2). The KLF4 reference sequence mRNA (NM_004235) consists of five exons and is transcribed from the negative strand of chromosome 9 from nucleotides 109,286,954-109,291,868 (human genome assembly version hg18; University of California Santa Cruz (UCSC) genome browser; Fig. 1).

To identify potential antisense transcripts from the KLF4 locus, the genomic region surrounding KLF4 was searched for spliced expressed sequence tags (ESTs) that mapped to the positive strand. Although it is normally difficult to determine the transcriptional orientation of ESTs, orientation can be determined by using splice site signatures of spliced ESTs. No spliced ESTs were found that overlapped KLF4, but the scan identified one antisense EST (DB461753) approximately 15 kb upstream of KLF4's annotated transcription start site (TSS). This EST was therefore chosen as a target candidate.

It was also decided to design short activating RNAs that targeted potential antisense transcripts from KLF4's promoter region. More specifically, the antisense sequence 500 nts upstream and downstream from KLF4's TSS (abbreviated KLF4_AS_TSS+/-500) was used as a second target candidate.

The aim was to design short RNAs for down-regulating the two candidate sequences. Candidate short RNAs should give effective inhibition of target sequences, and should ideally be as specific as possible such that potential off-target effects are minimized. Therefore the GPboost siRNA design algorithm was used to identify potential short RNAs for down-regulating the two candidate sequences. From the lists of predicted siRNA candidates, the two most promising non-overlapping siRNA target sites in the second exon of the antisense EST DB461753, and the most promising siRNA target site on each side of the KLF4 TSS within the antisense promoter sequence (KLF4_AS_TSS+/-500) were selected. The candidate siRNAs were selected based on predicted efficacy score from GPboost; absence of the sequence motifs aaaa, cccc, gggg, and tttt; moderate GC content of between 20% and 55%; and a Hamming distance of at least two to all potential off-target transcripts. Table 4 shows the resulting candidate short RNAs for activating KLF4 expression. The table shows both strands in a short RNA duplex, but the activating RNAs may also be administered as single stranded oligos (PMID: 12230974).

**Table 4**

| Activating small RNA (asRNA) candidates against KLF4. | | | | | |
|---|---|---|---|---|---|
| The table lists the two most promising siRNAs against the antisense EST DB461753 (IDs DB-1 and DB-2) and KLF4's promoter region (IDs Pr-1 and Pr-2). "Pos" is the target site start within the EST or the KLF4 promoter region; "Exon" is the target site's exon number; "Sense" shows the siRNAs' 19mer target site sequence; and "Antisense" shows the corresponding reverse-complementary sequence. The sense and antisense sequences plus 2 nt overhang sequences at their 3' ends (UU; not listed in the table) form the siRNA duplex candidates. | | | | | |
| ID | Target | Pos | Exon | Sense (passenger) | Antisense (guide) |
| DB-1 | DB461753 | 416 | 2 | GACCAUAUUUCUCUUGAAU | AUUCAAGAGAAAUAUGGUC |
| DB-2 | DB461753 | 313 | 2 | ACAAGGCUUCCAUUAAAGA | UCUUUAAUGGAAGCCUUGU |
| Pr-1 | AS TSS+/-500 | 514 | n/a | GCGCGUUCCUUACUUAUAA | UUAUAAGUAAGGAACGCGC |
| Pr-2 | AS TSS+/-500 | 26 | n/a | CUUCUUUGGAUUAAAUAUA | UAUAUUUAAUCCAAAGAAG |

### Candidate short RNAs activate KLF4 expression in CD34+ cells

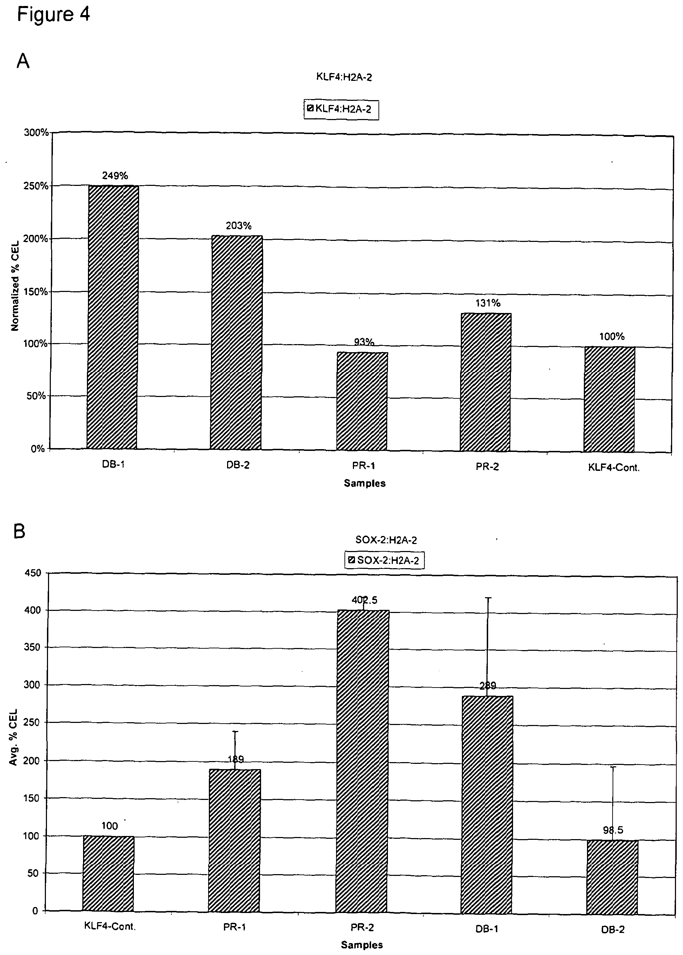

Cd34+ were treated with different Klf4 activating oligos [DB1, DB2, PR1 and PR2]. Klf4-DB1 seems to have a strong proliferative effect [Figure 3]. DB1 and DB2 have the highest Klf4 expression in cells [Figure 4a]. PR2, DB1 and PR1 show an increase in Sox2 expression [Figure 4b].

### Candidate short RNAs activate KLF4, c-Myc and nanog expression in Mesenchymal stem cells.

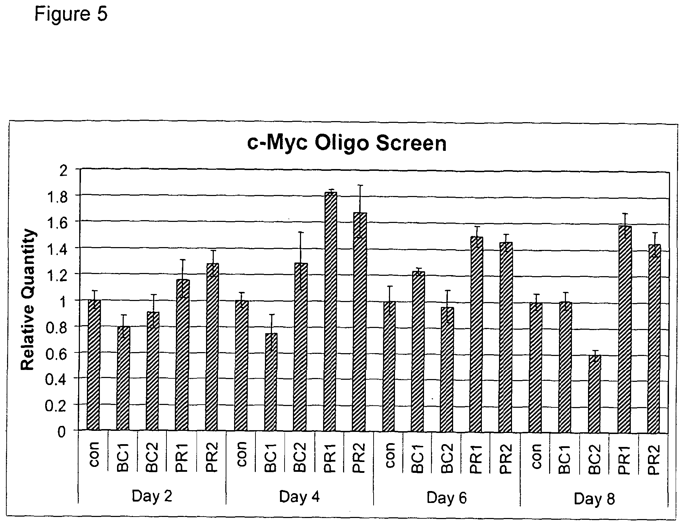

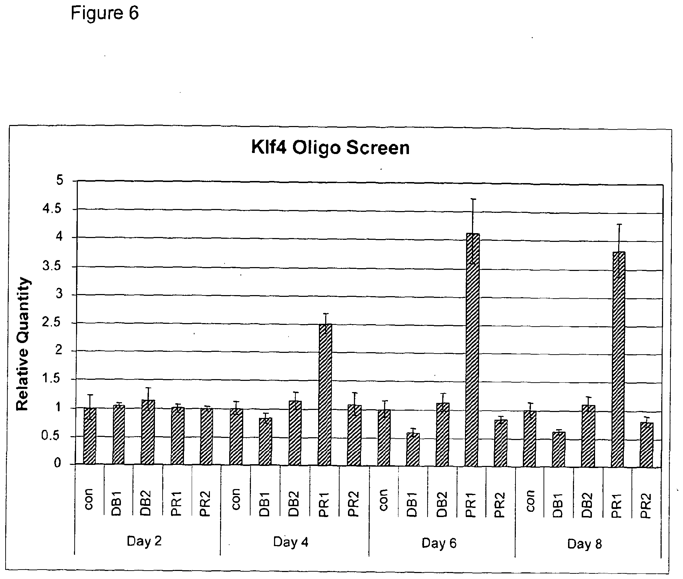

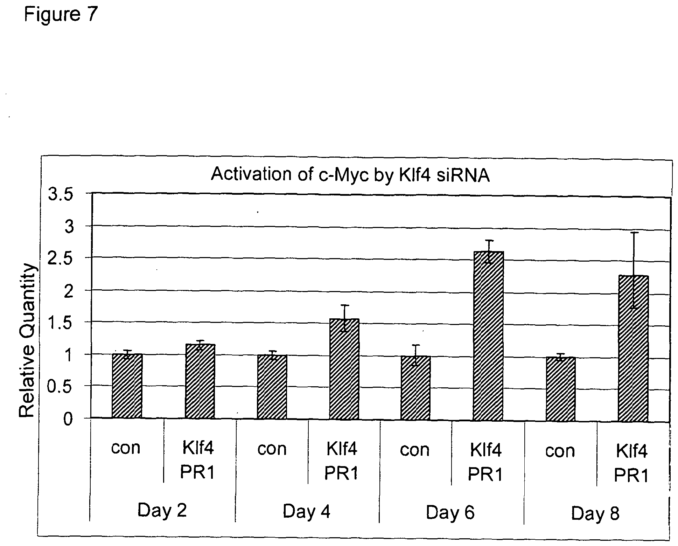

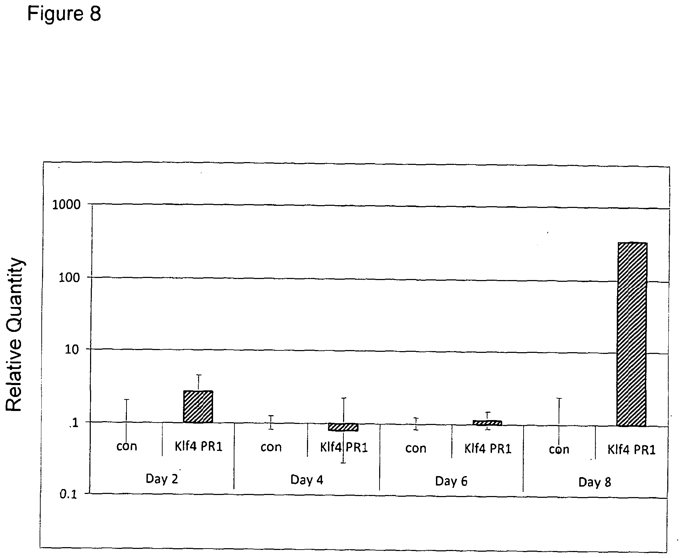

Using the same approach as for KLF4, oligos were designed for activating reprogramming factors MYC, POU5F1, and SOX2 (Table 5). MSCs were treated with c-Myc and Klf4 activating oligos. Figure 5 shows c-myc expression following administration of c-myc activating oligos. The highest effects were observed with PR1 and PR2 oligos. Figure 6, 7 and 8 show the Klf4, c-myc and nanog expression with klf4 activating oligos. Klf4-PR1 Oligo was shown to causes the highest expression of Klf4 as well as its down stream genes [c-myc and nanog].

**Table 5**

| Activating small RNA (asRNA) candidates against MYC. POU5F1. and SOX2. | | | | | | |
|---|---|---|---|---|---|---|
| The table lists the two most promising siRNAs against antisense ESTs and promoter regions of MYC, POU5F1, and SOX2. | | | | | | |
| Gene | ID | Target | Pos | Exon | Sense (passenger) | Antisense (guide) |
| MYC | BC-1 | BC042052 | 63 | 1 | GUGACUAUUCAACCGCAUA | UAUGCGGUUGAAUAGUCAC |
| MYC | BC-2 | BC042052 | 31 | 1 | GAGGAGUUACUGGAGGAAA | UUUCCUCCAGUAACUCCUC |
| MYC | Pr-1 | AS TSS+/- 500 | 787 | n/a | AGCAGUACUGUUUGACAAA | UUUGUCAAACAGUACUGCU |
| MYC | Pr-2 | AS TSS+/- 500 | 322 | n/a | GAAUUACUACAGCGAGUUA | UAACUCGCUGUAGUAAUUC |
| POU5F1 | BG-1 | BG203640 | 664 | 3 | UUUAAAUUCAAGAGAUCUA | UAGAUCUCUUGAAUUUAAA |
| POU5F1 | BG-2 | BG203640 | 622 | 2 | CGAGAACACCUGUCAAGUU | AACUUGACAGGUGUUCUCG |
| POU5F1 | Pr-1 | AS TSS+/- 500 | 940 | n/a | AUUCCUGUCCUCAAGAAAU | AUUUCUUGAGGACAGGAAU |
| POU5F1 | Pr-2 | AS TSS+/- 500 | 479 | n/a | UGAAAUGAGGGCUUGCGAA | UUCGCAAGCCCUCAUUUCA |
| SOX2 | BG-1 | BG220229 | 338 | 3 | AAAGGUCAUCUGACAUAAU | AUUAUGUCAGAUGACCUUU |
| SOX2 | BG-2 | BG220229 | 6 | 1 | CUGCUUUCCACCUAUGAAA | UUUCAUAGGUGGAAAGCAG |
| SOX2 | Pr-1 | AS TSS+/- 500 | 519 | n/a | GGGCUGUCAGGGAAUAAAU | AUUUAUUCCCUGACAGCCC |
| SOX2 | Pr-2 | AS TSS+/- 500 | 464 | n/a | UGACAACUCCUGAUACUUU | AAAGUAUCAGGAGUUGUCA |

### Discussion:

As shown in the Figures and discussed in the description of the figures, short RNAs targeting RNA transcripts which comprising sequences which are antisense to the target genes were shown to be functional and give strong up-regulation of the target. In particular those short RNAs which targeted RNA transcripts comprising a sequence which is antisense to the target genes' promoter regions were most effective.

The function of short RNAs may depend on the particular target cell type, i.e. as expected, it may be necessary for the target RNA transcript to be present in the cell being contacted in order for the short RNA molecule to have an effect. As shown, short RNAs such as KLF4 RNAs DB-1 and DB-2, showed strong up-regulation of KLF4 in OmniCytes but had less effect in MSCs. This is likely because the target transcript has cell-type specific expression. The results also show that short RNAs which up-regulate KLF4 also result in up-regulation of KLF4's down-stream targets Nanog and c-Myc. This is according to the established model where KLF4 transcriptionally regulates Nanog and c-Myc and shows that the activating RNAs function as intended.

### Example 2

The following saRNA molecules were designed according to the method of the present disclosure by a) obtaining the sequence of the target gene in the region 500 nucleotides upstream of the transcription start site to 500 nucleotides downstream of the transcription start site, b) determining the reverse complementary RNA sequence to the sequence of step a) and c) designing saRNAs which are complementary to a region of the sequence determined in b).

**Table 6**

| Activating small RNA (saRNA) candidates against BCL2 and IL8. | | | |
|---|---|---|---|
| Gene | ID | Sense (passenger) | Antisense (guide) |
| BCL2 | PR1 | GAGGAUUUCCAGAUCGAUUUU | AAUCGAUCUGGAAAUCCUCUU |
| BCL2 | PR2 | UCAGCACUCUCCAGUUAUAUU | UAUAACUGGAGAGUGCUGAUU |
| BCL2 | PR3 | GCAGGAAUCCUCUUCUGAUUU | AUCAGAAGAGGAUUCCUGCUU |
| BCL2 | PR4 | GCAGAAGUCCUGUGAUGUUUU | AACAUCACAGGACUUCUGCUU |
| IL8 | PR1 | UUCAUUAUGUCAGAGGAAAUU | UUUCCUCUGACAUAAUGAAUU |
| IL8 | PR2 | CGCUGUAGGUCAGAAAGAUUU | AUCUUUCUGACCUACAGCGUU |

The saRNA molecules were produced and transfected into either Omnicytes or somatic cells (HepG2 & SHSY5Y). The effect on the expression of the target gene was assessed by quantifying the mRNA levels of the target gene by RT-PCR.

### Transfection of saRNA oligonucleotides:

The saRNA oligonucleotide pairs (Sense and Antisense, shown in Table 6 above) were first annealed using 50mM Tris-HCl, pH8.0, 100mM NaCl and 5mM EDTA following a denaturation step at 90°C followed by a gradual anneal step to room temperature. 150ng of paired saRNA was then transfected into cells using Nanofectamine (PAA, UK) following the manufacturer's instructions. Cells were then harvested 24 hours following transfection for rtPCR analysis

### Isolation of total RNA for semi-quantitative rtPCR:

All total RNA extraction was carried out using the RNAqueous-Micro kit (Ambion, UK) following the manufacturer's instructions. Briefly, the cells were gently centrifuged followed by 3 pulses of sonication at Output 3 in Lysis buffer (Ambion, UK). The cell lysates were then processed through an RNA binding column, followed by multiple washes and elution. The total RNA isolated was quantified by a Nanodrop 2000 spectrophotometer. 500ng of total RNA was reversed transcribed using One Step RT-PCR (Qiagen, Germany) following the manufacturer's instructions. Expression for the target genes were performed by reverse-transcrption PCR (rtPCR) using their respective primer pairs. mRNA levels are expressed relative to relative to the house keeping gene actin.

### Results:

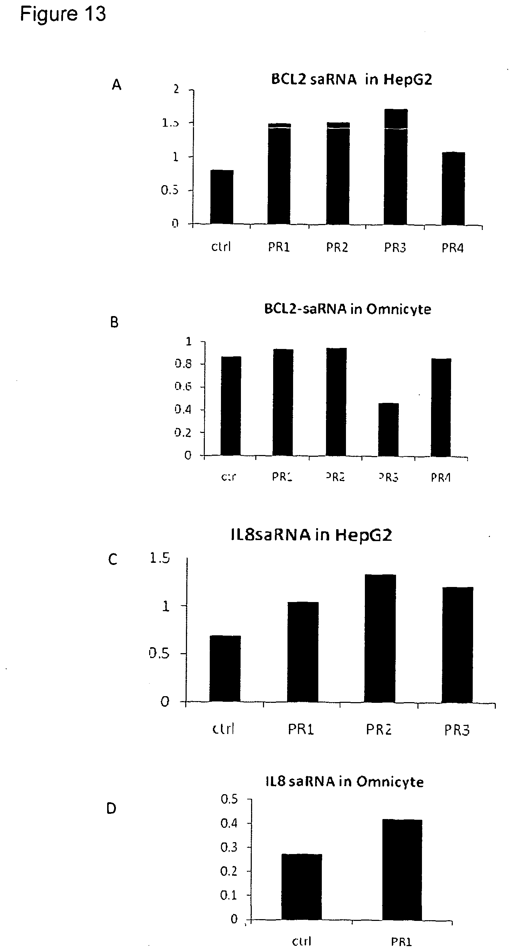

The results are shown in Figure 13. The mRNA profile of cells transfected with saRNA demonstrates that the target mRNA transcripts increased relative to the control.

## Claims

1. A method of producing a single-stranded, and not double-stranded, short RNA molecule to increase the expression of a target gene in a cell through the down-regulation of a non-coding RNA transcript, said method comprising the steps of:
a) obtaining the nucleotide sequence of the coding strand of the target gene between 200 nucleotides upstream of the gene's transcription start site and 200 nucleotides downstream of the gene's transcription start site;
b) determining the reverse complementary RNA sequence to the nucleotide sequence determined in step a);
c) designing a single-stranded short RNA molecule which is from 16 nucleotides to 30 nucleotides in length and which is the reverse complement or has at least 95% sequence identity with the reverse complement of a region of the sequence determined in step b); and
d) synthesising the single-stranded short RNA molecule, and not a double-stranded short RNA molecule, wherein the synthesised single-stranded short RNA molecule comprises non-standard nucleotides;
wherein said method does not include a step in which the existence of said non-coding RNA transcript is determined.

2. The method of claim 1, wherein the target gene is a pluripotency-inducing gene.

3. The method of claim 1 or claim 2, wherein the region defined in c) includes the reverse complement of the gene's transcription start site.

4. The method of any one of the preceding claims, wherein the short RNA molecule is 21 nucleotides in length.

5. An in vitro method of increasing the expression of a target gene in a cell through the down-regulation of a non-coding RNA transcript, said method comprising the steps of:
a) obtaining the nucleotide sequence of the coding strand of the target gene between 200 nucleotides upstream of the gene's transcription start site and 200 nucleotides downstream of the gene's transcription start site;
b) determining the reverse complementary RNA sequence to the nucleotide sequence determined in step a);
c) designing a single-stranded short RNA molecule which is from 16 nucleotides to 30 nucleotides in length and which is the reverse complement or has at least 95% sequence identity with the reverse complement of a region of the sequence determined in step b); and
d) contacting the cell with said single-stranded short RNA molecule,
wherein said single-stranded short RNA molecule comprises non-standard nucleotides, wherein said single-stranded short RNA molecule down-regulates said non-coding RNA transcript,
wherein said method does not include a step in which the existence of said non-coding RNA transcript is determined.

6. One or more computer-readable media comprising computer-executable instructions to instruct a computing system to:
a) receive a nucleotide sequence of a coding strand of a target gene between 200 nucleotides upstream of the gene's transcription start site and 200 nucleotides downstream of the gene's transcription start site;
b) determine a reverse complementary RNA sequence to the nucleotide sequence received in step a); and
c) output information to construct a single-stranded short RNA molecule designed to increase expression of the target gene in a cell through the down-regulation of a non-coding RNA transcript wherein the short RNA molecule is from 16 nucleotides to 30 nucleotides in length and is the reverse complement or has at least 95% sequence identity with the reverse complement of a region of the sequence determined in step b); wherein the instructions do not comprise instructions to call for determining existence of the non-coding RNA transcript.

7. The one or more computer-readable media comprising computer-executable instructions to instruct a computing system of claim 6, further comprising instructions to output the information to construct a short RNA molecule onto a computer-readable medium.

## Patentansprüche

1. Verfahren zum Herstellen eines einzelsträngigen, und nicht doppelsträngigen, kurzen RNS-Moleküls, um die Expression eines Zielgens in einer Zelle durch die Herunterregulierung eines nicht-kodierenden RNS-Transkripts zu erhöhen, wobei das Verfahren die Schritte umfasst des:
a) Erhaltens der Nukleotidsequenz des kodierenden Strangs des Zielgens, zwischen 200 Nukleotide strangaufwärts der Transkriptions-Startstelle des Gens und 200 Nukleotide strangabwärts der Transkriptions-Startstelle des Gens;
b) Bestimmens der revers-komplementären RNS-Sequenz zu der in Schritt a) bestimmten Nukleotidsequenz;
c) Ausgestaltens eines einzelsträngigen, kurzen RNS-Moleküls, welches von 16 Nukleotide bis 30 Nukleotide lang ist, und welches das Revers-Komplement ist oder mindestens 95% Sequenzidentität hat mit dem Revers-Komplement einer Region der in Schritt b) bestimmten Sequenz; und des
d) Synthetisierens des einzelsträngigen, kurzen RNS-Moleküls, und nicht eines doppelsträngigen, kurzen RNS-Moleküls, wobei das synthetisierte einzelsträngige, kurze RNS-Molekül nicht-standardmäßige Nukleotide umfasst;
wobei das Verfahren keinen Schritt einschließt, in dem die Existenz des nicht-kodierenden RNS-Transkripts bestimmt wird.

2. Verfahren gemäß Anspruch 1, wobei das Zielgen ein Pluripotenz-induzierendes Gen ist.

3. Verfahren gemäß Anspruch 1 oder Anspruch 2, wobei die in c) definierte Region das Revers-Komplement der Transkriptions-Startstelle des Gens einschließt.

4. Verfahren gemäß irgendeinem der vorangegangenen Ansprüche, wobei das kurze RNS-Molekül 21 Nukleotide lang ist.

5. In vitro-Verfahren zum Erhöhen der Expression eines Zielgens in einer Zelle durch die Herunterregulierung eines nicht-kodierenden RNS-Transkripts, wobei das Verfahren die Schritte umfasst des:
a) Erhaltens der Nukleotidsequenz des kodierenden Strangs des Zielgens, zwischen 200 Nukleotide strangaufwärts der Transkriptions-Startstelle des Gens und 200 Nukleotide strangabwärts der Transkriptions-Startstelle des Gens;
b) Bestimmens der revers-komplementären RNS-Sequenz zu der in Schritt a) bestimmten Nukleotidsequenz;
c) Ausgestalten eines einzelsträngigen, kurzen RNS-Moleküls, welches von 16 Nukleotide bis 30 Nukleotide lang ist, und welches das Revers-Komplement ist oder mindestens 95% Sequenzidentität hat mit dem Revers-Komplement einer Region der in Schritt b) bestimmten Sequenz; und des
d) in Kontakt Bringens der Zelle mit dem einzelsträngigen, kurzen RNS-Molekül, wobei das einzelsträngige, kurze RNS-Molekül nicht-standardmäßige Nukleotide umfasst, wobei das einzelsträngige, kurze RNS-Molekül das nicht-kodierende RNS-Transkript herunterreguliert,
wobei das Verfahren keinen Schritt einschließt, in dem die Existenz des nicht-kodierenden RNS-Transkripts bestimmt wird.

6. Ein oder mehrere computer-lesbare(s) Medium/Medien, umfassend computer-ausführbare Anweisungen, um ein Computersystem (computing system) anzuweisen:
a) eine Nukleotidsequenz eines kodierenden Strangs eines Zielgens, zwischen 200 Nukleotide strangaufwärts der Transkriptions-Startstelle des Gens und 200 Nukleotide strangabwärts der Transkriptions-Startstelle des Gens, zu empfangen;
b) eine revers-komplementäre RNS-Sequenz zu der in Schritt a) empfangenen Nukleotidsequenz zu bestimmen; und
c) Information(en) zum Erstellen eines einzelsträngigen, kurzen RNS-Moleküls auszugeben, das ausgestaltet ist, die Expression des Zielgens in einer Zelle durch die Herunterregulierung eines nicht-kodierenden RNS-Transkripts zu erhöhen, wobei das kurze RNS-Molekül von 16 Nukleotide bis 30 Nukleotide lang ist, und das Revers-Komplement ist oder mindestens 95% Sequenzidentität hat mit dem Revers-Komplement einer Region der in Schritt b) bestimmten Sequenz;
wobei die Anweisungen keine Anweisungen umfassen, das Bestimmen der Existenz des nicht-kodierenden RNS-Transkripts zu veranlassen.

7. Das eine oder die mehreren computer-lesbare(n) Medium/Medien gemäß Anspruch 6, umfassend computer-ausführbare Anweisungen, um ein Computersystem (computing system) anzuweisen, weiter umfassend Anweisungen, die Information(en) zum Erstellen eines kurzen RNS-Moleküls auf ein computer-lesbares Medium auszugeben.

## Revendications

1. Procédé de fabrication d'une molécule d'ARN court simple brin, et non double-brin, pour augmenter l'expression d'un gène cible dans une cellule via la régulation vers le bas d'un transcrit d'ARN non codant, ledit procédé comprenant les étapes :
a) d'obtention de la séquence nucléotidique du brin codant du gène cible entre 200 nucléotides en amont du site de départ de transcription du gène et 200 nucléotides en aval du site de départ de transcription du gène ;
b) de détermination de la séquence d'ARN complémentaire inverse à la séquence nucléotidique déterminée à l'étape a) ;
c) de conception d'une molécule d'ARN court simple brin qui a une longueur de 16 nucléotides à 30 nucléotides et qui est le complément inverse ou présente au moins 95 % d'identité de séquence avec le complément inverse d'une région de la séquence déterminée à l'étape b) ; et
d) de synthèse de la molécule d'ARN court simple brin, et pas d'une molécule d'ARN court double brin, dans lequel la molécule d'ARN court simple brin synthétisée comprend des nucléotides non standards;
dans lequel ledit procédé ne comprend pas une étape dans laquelle l'existence dudit transcrit d'ARN non codant est déterminée.

2. Procédé selon la revendication 1, dans lequel le gène cible est un gène inducteur de pluripotence.

3. Procédé selon la revendication 1 ou la revendication 2, dans lequel la région définie à l'étape c) comprend le complément inverse du site de départ de transcription du gène.

4. Procédé selon l'une quelconque des revendications précédentes, dans lequel la molécule d'ARN court a une longueur de 21 nucléotides.

5. Procédé in vitro pour augmenter l'expression d'un gène cible dans une cellule par la régulation vers le bas d'un transcrit d'ARN non codant, ledit procédé comprenant les étapes :
a) d'obtention de la séquence nucléotidique du brin codant du gène cible entre 200 nucléotides en amont du site de départ de transcription du gène et 200 nucléotides en aval du site de départ de transcription du gène ;
b) de détermination de la séquence d'ARN complémentaire inverse à la séquence nucléotidique déterminée à l'étape a) ;
c) de conception d'une molécule d'ARN court simple brin qui a une longueur de 16 nucléotides à 30 nucléotides et qui est le complément inverse ou présente au moins 95 % d'identité de séquence avec le complément inverse d'une région de la séquence déterminée à l'étape b) ; et
d) la mise en contact de la cellule avec ladite molécule d'ARN court simple brin,
dans lequel ladite molécule d'ARN court simple brin comprend des nucléotides non standards,
dans lequel ladite molécule d'ARN court simple brin régule vers le bas ledit transcrit d'ARN non codant,
dans lequel ledit procédé ne comprend pas une étape dans laquelle l'existence dudit transcrit d'ARN non codant est déterminée.

6. Support(s) lisible(s) par ordinateur comprenant des instructions exécutables par ordinateur pour donner des instructions à un système informatique pour :
a) recevoir une séquence nucléotidique d'un brin codant d'un gène cible entre 200 nucléotides en amont du site de départ de transcription du gène et 200 nucléotides en aval du site de départ de transcription du gène;
b) déterminer une séquence d'ARN complémentaire inverse à la séquence nucléotidique reçue à l'étape a) ; et
c) délivrer en sortie des informations pour construire une molécule d'ARN court simple brin conçue pour augmenter une expression du gène cible dans une cellule par la régulation vers le bas d'un transcrit d'ARN non codant dans le(s)quel(s) la molécule d'ARN court a une longueur de 16 nucléotides à 30 nucléotides et est le complément inverse ou a au moins 95% d'identité de séquence avec le complément inverse d'une région de la séquence déterminée à l'étape b) ;
dans le(s)quel(s) les instructions ne comprennent pas d'instructions pour amener à déterminer l'existence du transcrit d'ARN non codant.

7. Support(s) lisible(s) par ordinateur comprenant des instructions exécutables par ordinateur pour donner des instructions à un système informatique selon la revendication 6, comprenant en outre des instructions pour délivrer en sortie les informations pour construire une molécule d'ARN court sur un support lisible par ordinateur.
